(19) Europäisches Patentamt
European Patent Office
Office européen des brevets

(11) EP 3 035 206 A1

(12) **EUROPEAN PATENT APPLICATION**

(43) Date of publication:
22.06.2016 Bulletin 2016/25

(51) Int Cl.:
G06F 17/18 (2006.01)    A61B 5/145 (2006.01)
A61B 5/1455 (2006.01)    A61B 5/1495 (2006.01)

(21) Application number: 15188542.3

(22) Date of filing: 06.10.2015

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 08.10.2014 JP 2014207166

(71) Applicant: Seiko Epson Corporation
Tokyo 163 (JP)

(72) Inventors:
• KURASAWA, Hikaru
Nagano, 392-8502 (JP)
• ARAI, Yoshifumi
Nagano, 392-8502 (JP)

(74) Representative: Hoffmann Eitle
Patent- und Rechtsanwälte PartmbB
Arabellastraße 30
81925 München (DE)

(54) **CALIBRATION CURVE GENERATION DEVICE, TARGET COMPONENT CALIBRATION DEVICE, ELECTRONIC DEVICE, AND GLUCOSE CONCENTRATION CALIBRATION DEVICE**

(57)    A calibration curve generation device includes an estimation unit that estimates a plurality of independent components or main components constituting observation spectral data of a plurality of samples and a regression formula calculation unit that acquires a regression formula of a calibration curve based on glucose concentration of the plurality of samples and a mixing coefficient of the independent components or the main components in the observation spectral data for each of the samples. The estimation unit selects a component waveform signal having a peak in a wavelength selected in advance as the independent components or the main components.

FIG. 7

**Description**

<u>BACKGROUND</u>

1. Technical Field

The present invention relates to a technique of acquiring the content of a target component related to a subject from measurement data of the subject.

2. Related Art

JP-A-2010-66280 describes a technique of measuring an absorption spectrum of an organism using near-infrared light and determining glucose concentration according to the absorption spectrum. In the technique of the related art, three continuous wavelength bandwidths of a first wavelength bandwidth (1550 nm to 1650 nm) for measuring absorption derived from an OH group of glucose molecules, a second wavelength bandwidth (1480 nm to 1650 nm) for measuring absorption derived from an NH group of organism components, and a third wavelength bandwidth (1650 nm to 1880 nm) for measuring absorption derived from a CH group are selected from a wavelength range of 1480 nm to 1880 nm with less influence of absorption of water and continuously measure an absorption spectrum. In addition, the quantity of glucose is determined by performing PLS regression analysis using the measured absorption spectrum as an explanatory variable and the glucose concentration as a target variable. Moreover, in the technique in the related art, improvement of precision is attempted using absorption spectrum data in a specific wavelength bandwidth (1480 nm to 1880 nm) considered to include a plurality of pieces of information related to glucose from among the wavelength bandwidth of near-infrared light and using not one specific wavelength but three to five wavelengths from the absorption spectrum in order to analyze overlapping information.

However, in the technique of the related art, although the wavelength bandwidth including a plurality of pieces of information related to glucose is selected, the fact that information related to other organism components overlap each other and is included therein is not changed and thus it is difficult to separate and extract the information related to glucose from other pieces of information in principle. Accordingly, there is a problem in that the calibration precision is not sufficiently obtained in some cases.

<u>SUMMARY</u>

An advantage of some aspects of the invention is to solve at least a part of the problems described above, and the invention can be implemented as the following forms or application examples.

(1) A first aspect of the invention provides a calibration curve generation device which generates a calibration curve used for deriving glucose concentration of a subject from observation spectral data of the subject including glucose. The calibration curve generation device includes an estimation unit that estimates a plurality of independent components or main components constituting observation spectral data of a plurality of samples and a regression formula calculation unit that acquires a regression formula of the calibration curve based on the glucose concentration of the plurality of samples and a mixing coefficient of the independent components or the main components in the observation spectral data for each of the samples. The estimation unit selects a component waveform signal having a peak in a wavelength selected in advance as the independent components or the main components.

According to this calibration curve generation device, since the independent component or the main component having a peak in the wavelength selected in advance as a wavelength corresponding to glucose is selected and a regression formula is calculated using the independent component or the main component, it is possible to obtain a regression formula of a calibration curve with high calibration precision in regard to the glucose concentration.

In one embodiment, the calibration curve generation device includes a sample observation data acquisition unit that acquires the observation spectral data related to a plurality of samples of the subject; a glucose concentration acquisition unit that acquires the glucose concentration related to each of the samples; an estimation unit that estimates a plurality of independent components or main components when the observation spectral data for each of the samples is divided to the plurality of independent components or the main components; and a regression formula calculation unit that acquires a regression formula of the calibration curve based on the glucose concentrations of the plurality of samples and the mixing coefficient of the independent components or the main components in the observation spectral data for each of the samples, in which the estimation unit selects a component having a peak in a wavelength selected in advance as the independent components or the main components.

According to this calibration curve generation device, since the independent component or the main component having a peak in the wavelength selected in advance as a wavelength corresponding to glucose is selected and a regression formula is calculated using the independent component or the main component, it is possible to obtain a regression formula of a calibration curve with high calibration precision in regard to the glucose concentration.

(2) In the calibration curve generation device, the wavelength selected in advance may be one of (i) at least one wavelength between wavelengths of $940 \pm 30$ nm and $1025 \pm 30$ nm and (ii) at least one wavelength between wavelengths of $1135 \pm 30$ nm and $1210 \pm 30$ nm.

According to this configuration, since at least one wavelength between wavelengths of $940 \pm 30$ nm and $1025 \pm 30$ nm or at least one wavelength between wavelengths of $1135 \pm 30$ nm and $1210 \pm 30$ nm is selected as the wavelength corresponding to glucose, it is possible to obtain a regression formula of a calibration curve with high calibration precision in regard to the glucose concentration.

(3) In the calibration curve generation device, the estimation unit may include an independent component matrix calculation unit that calculates an independent component matrix including the plurality of independent components and an independent component selection unit that selects an independent component having a peak in the wavelength selected in advance from the independent component matrix.

According to this device, the independent component having a peak in the wavelength corresponding to glucose can be automatically selected by the independent component selection unit.

(4) A second aspect of the invention provides a target component calibration device which acquires glucose concentration related to a subject including glucose as a target component. The target component calibration device includes a mixing coefficient calculation unit that acquires a mixing coefficient with respect to the glucose related to the subject based on observation spectral data and calibration data related to the subject and a target component amount calculation unit that calculates the glucose concentration based on a single regression formula indicating a relationship between the mixing coefficient and the glucose concentration corresponding to the glucose and the mixing coefficient acquired by the mixing coefficient calculation unit. The mixing coefficient calculation unit uses a component having a peak in the wavelength selected in advance as the independent component or the main component with respect to the glucose.

According to this target component calibration device, since the calibration of glucose concentration is performed using the independent component or the main component having a peak in the wavelength selected in advance as a wavelength corresponding to glucose, it is possible to improve calibration precision in regard to the glucose concentration.

In one embodiment, the target component calibration device includes a subject observation data acquisition unit that acquires the observation spectral data related to the subject; a calibration data acquisition unit that acquires calibration data including an independent component or a main component corresponding to the glucose and a single regression formula for calibration; a mixing coefficient calculation unit that acquires a mixing coefficient with respect to the glucose related to the subject based on the observation data and the calibration data related to the subject; and a target component amount calculation unit that calculates the glucose concentration based on the single regression formula indicating a relationship between the mixing coefficient and the glucose concentration corresponding to the glucose and the mixing coefficient acquired by the mixing coefficient calculation unit. The mixing coefficient calculation unit uses a component having a peak in the wavelength selected in advance as the independent component or the main component with respect to the glucose.

According to this target component calibration device, since the calibration of glucose concentration is performed using the independent component or the main component having a peak in the wavelength selected in advance as a wavelength corresponding to glucose, it is possible to improve calibration precision in regard to the glucose concentration.

(5) In the target component calibration device, the wavelength selected in advance may be one of (i) at least one wavelength between wavelengths of $940 \pm 30$ nm and $1025 \pm 30$ nm and (ii) at least one wavelength between wavelengths of $1135 \pm 30$ nm and $1210 \pm 30$ nm.

According to this configuration, since at least one wavelength between wavelengths of $940 \pm 30$ nm and $1025 \pm 30$ nm or at least one wavelength between wavelengths of $1135 \pm 30$ nm and $1210 \pm 30$ nm is selected as the wavelength corresponding to glucose, it is possible to improve calibration precision in regard to the glucose concentration.

The invention can be implemented using various aspects other than the above-described aspects. For example, the invention can be implemented using an electronic device including the above-described device, a computer program implementing functions of respective units of the above-described device, and a recording medium which stores a computer program and is not temporary (non-transitory storage medium).

BRIEF DESCRIPTION OF THE DRAWINGS

The invention will be described with reference to the accompanying drawings, wherein like numbers reference like elements.

Fig. 1 is an explanatory diagram showing an outline of a calibration curve generation process using independent component analysis.

Fig. 2 is an explanatory diagram showing an outline of a calibration process of a target component.

Fig. 3 is a flowchart of the calibration curve generation process for a blood sugar level.

Fig. 4A is an explanatory diagram showing an example of an independent component for calibration of a blood sugar level.

Fig. 4B is an explanatory diagram showing an example of an independent component for calibration of a blood sugar level.

Fig. 5 is a flowchart of the calibration curve generation process.

Fig. 6 is an explanatory diagram showing a computer used in the calibration curve generation process.

Fig. 7 is a functional block diagram of a device used in the calibration curve generation process.

Fig. 8 is a functional block diagram showing an example of an internal configuration of an independent component matrix calculation unit.

Fig. 9 is an explanatory diagram schematically showing a measurement dataset DS1.

Fig. 10 is a flowchart showing a mixing coefficient estimation process.

Fig. 11 is an explanatory diagram describing an estimated mixed matrix $\wedge A$.

Fig. 12 is a flowchart showing a calculation process of a regression formula.

Fig. 13 is a functional block diagram of a device to be used in the calibration process of the target component.

Fig. 14 is a flowchart showing the calibration process of the target component.

DESCRIPTION OF EXEMPLARY EMBODIMENTS

Hereinafter, embodiments of the invention will be described in the following order.

A. Outline of calibration curve generation process and calibration process:
B. Calibration curve generation method for blood sugar level:
C. Calibration curve generation method:
D. Calibration method of target component:
E. Various algorithms and influence thereof:
F. Modification Examples:

In the present embodiment, the following abbreviations are used.

- ICA: independent component analysis
- SNV: standard normal variate transformation
- PNS: project on null space
- PCA: principal component analysis
- FA: factor analysis

A. Outline of calibration curve generation process and calibration process:

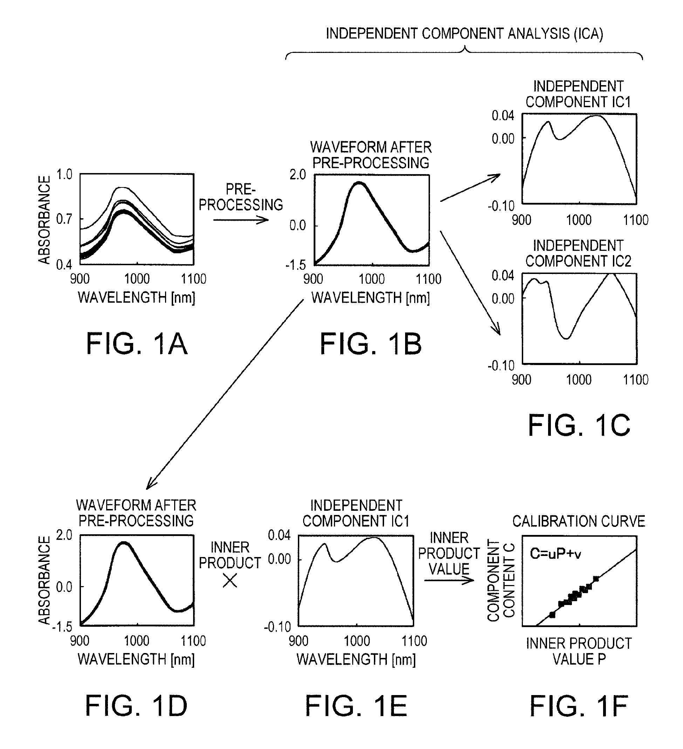

Fig. 1 is an explanatory diagram showing the outline of a calibration curve generation process using independent component analysis. Fig. 1 (A) shows an example of observation data (also referred to as "measurement data") related to a plurality of samples. The observation data relates to spectral absorbance and, for example, can be obtained through spectrometry of samples including a plurality of chemical components such as glucose. As a plurality of samples used in the calibration curve generation process, samples in which the content of target components (for example, glucose) is known are used. Alternatively, the content of the target components included in the plurality of samples may be measured by an analysis device.

When a calibration curve is generated, first, fluctuation or noise included in the observation data is reduced by performing pre-processing on the observation data (Fig. 1(B)). As the pre-processing, for example, first pre-processing that includes normalizing the observation data and second pre-processing that includes whitening are performed. In the first pre-processing, in order to reduce the influence of various fluctuation factors (the state of a sample, a change in the measurement environment, and the like) in the observation data, it is preferable to perform project on null space. Next, by performing independent component analysis processing on observation data after the pre-processing is finished, a plurality of independent components IC1, IC2, ... , and the like (Fig. 1(C)) are obtained. These independent components IC1, IC2, ... , and the like are data corresponding to respective material components included in each sample and components statistically independent from each other. The observation data of each sample can be reproduced as a linear combination of these independent components IC1, IC2, ... , and the like. In Fig. 1(C), only two independent components IC1 and IC2 are shown, but the number of independent components is appropriately set to an arbitrary number of 2 or greater. Further, in the description of embodiments, the term "target component" indicates a material or a chemical component included in a sample and the term "independent component" indicates data having a data length which is the same as that of observation data of the sample.

Next, as shown in (D) to (F) in Fig. 1, the inner product between the observation data after the pre-processing is performed and an independent component (for example, IC1) is calculated. The observation data of Fig. 1(D) is the same as the data of Fig. 1(B). When the inner product between one piece of observation data and one independent component IC1 is acquired, one inner product value in regard to the observation data is obtained. Therefore, when the inner product between a plurality of pieces of observation data and the same independent component IC1 is calculated, a plurality of inner product values are obtained with respect to the same independent component IC1 in regard to the plurality of samples. Fig. 1(F) is a diagram in which an inner product value p in regard to the plurality of samples is set as the horizontal axis and a known content C of a target component included in the plurality of samples is set as the vertical axis and then plotted. If the independent component IC1 used in the inner product is an independent component corresponding to a target component, as shown in Fig. 1(F), the inner product value p is strongly correlated with the content C of the target component of each sample. Here, an independent component showing the strongest correlation among the plurality of independent components IC1, IC2, ..., and the like obtained in Fig. 1(C) can be selected as an independent component corresponding to a target component. In the example of Fig. 1, the independent component IC1 is the independent component corresponding to a target component (for example, glucose) of calibration. The calibration curve is expressed as a straight line given by a simple regression formula "C = uP + v" of the plot in Fig. 1(F). Moreover, since the inner product value P is a value proportional to the content of the independent component IC1 in each sample, the inner product value P is also referred to as a "mixing coefficient."

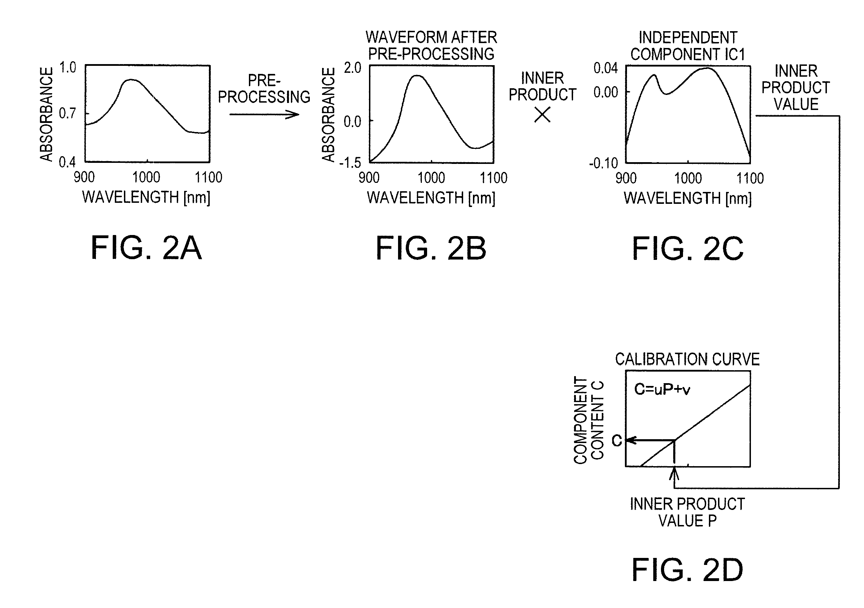

Fig. 2 is an explanatory diagram showing the outline of a calibration process of the target component using a calibration curve. The calibration process is performed using the independent component IC1 (Fig. 1(E)) of the target component obtained by the calibration curve generation process shown in (A) to (F) in Fig. 1 and the calibration curve (Fig. 1(F)). In the calibration process, first, observation data of a sample in which the content of a target component is unknown is obtained (Fig. 2(A)). Next, the pre-processing is performed on the observation data (Fig. 2(B)). It is preferable that the pre-processing is performed in the same manner as the pre-processing used at the time of generating a calibration curve. Further, the inner product value P in regard to the observation data is calculated by acquiring the inner product between the observation data after the pre-processing and the independent component IC1 (Fig. 2(B)). When the inner product value P is applied to the calibration curve (Fig. 2(D)), the content C of a target component can be determined. Moreover, the calibration curve generation process of Fig. 1 and the calibration process of Fig. 2 will be described below in detail.

B. Calibration curve generation method for blood sugar level:

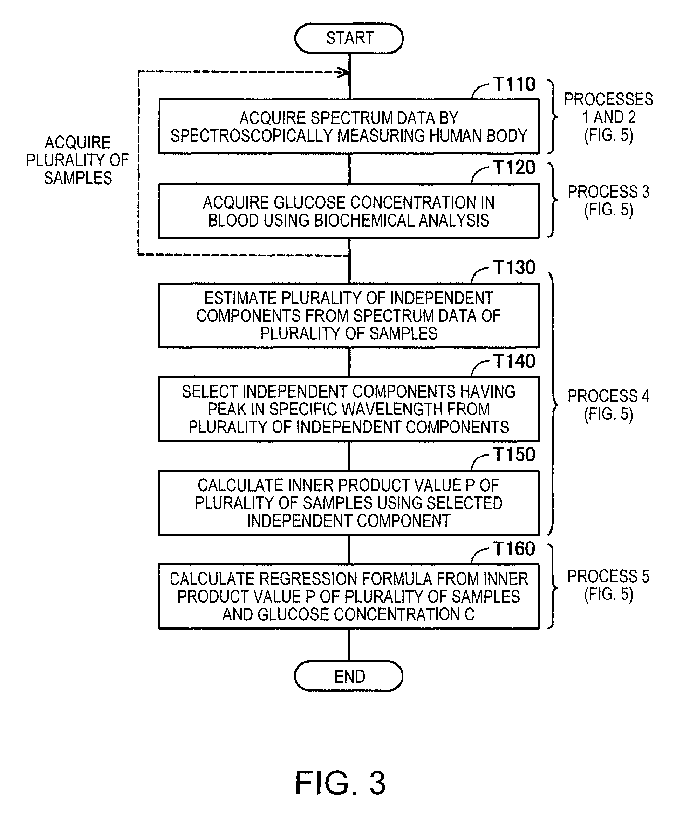

Fig. 3 is a flowchart of the calibration curve generation process for a blood sugar level. In the example, the subject is a human body (for example, a hand) and the target component is glucose (blood sugar). It is known that the blood sugar level is glucose concentration in blood. In addition, a correspondence relation with the processes 1 to 5 in Fig. 3 which will be described later is shown on the right side of Steps T110 to T160 in Fig. 3.

In Step T110, spectrum data (also referred to as "measurement spectral data" or "measurement data") is acquired by spectroscopically measuring a human body. In Step T120, the glucose concentration in blood is accurately acquired by collecting the blood from the same human body as that in Step T110 and performing biochemical analysis. By repeatedly performing the processes of Steps T110 and T120 plural times, spectrum data and the glucose concentration related to a plurality of samples are acquired.

In Step T130, a plurality of independent components are estimated from the spectrum data of the plurality of samples by following the procedures shown in (A) to (C) in Fig. 1. In Step T140, an independent component having a peak in a specific wavelength selected in advance is selected from the plurality of independent components obtained in this manner. The specific wavelength is selected in advance as a preferable wavelength corresponding to glucose. As the specific wavelength, any one of the wavelength described below can be used.

(i) In a case of using spectrum data of a wavelength bandwidth of 900 nm to 1100 nm:

At least one wavelength between wavelengths of $940 \pm \alpha$ nm and $1025 \pm \alpha$ nm

(ii) In a case of using spectrum data of a wavelength bandwidth of 1100 nm to 1250 nm:

At least one wavelength between wavelengths of $1135 \pm \alpha$ nm and $1210 \pm \alpha$ nm.

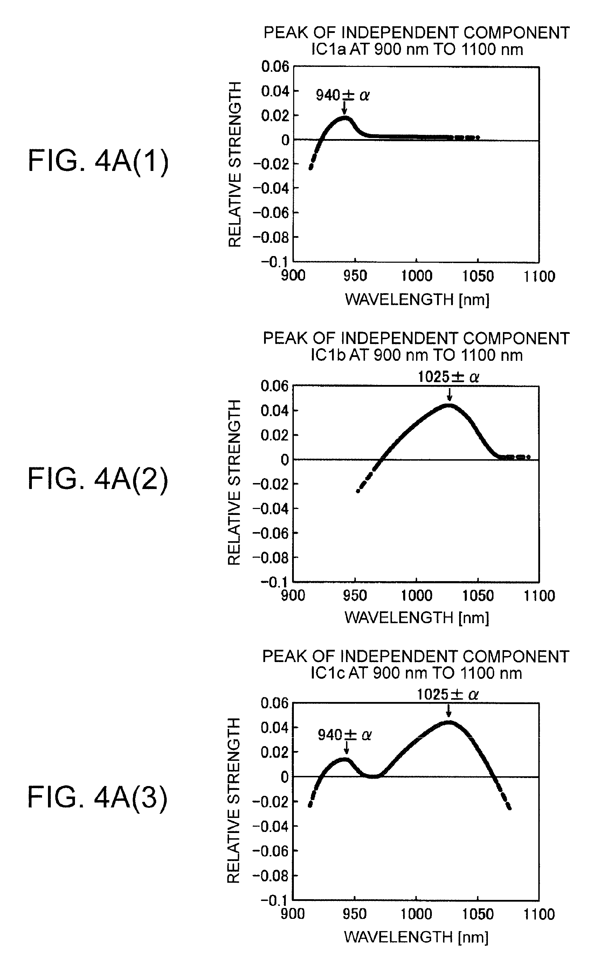

Fig. 4A shows an example of the preferable independent component in a case where spectrum data having a wavelength bandwidth of 900 nm to 1100 nm is used for calibration. A first independent component IC1a shown in (1) of Fig. 4A is an independent component which has a peak at $940 \pm \alpha$ [nm] and does not have a peak at $1025 \pm \alpha$ [nm].

A second independent component IC1b shown in (2) of Fig. 4A is an independent component which does not have a peak at $940 \pm \alpha$ [nm] and has a peak at $1025 \pm \alpha$ [nm]. A third independent component IC2c shown in (3) of Fig. 4A is an independent component which has a peak at both of $940 \pm \alpha$ [nm] and $1025 \pm \alpha$ [nm]. $\alpha$ represents a value for restricting the range of a peak specific to the glucose. As the value of $\alpha$, 30 is preferable and 20 is more preferable. Moreover, the third independent component IC1c corresponds to the sum of the first independent component IC1a and the second independent component IC1b.

In a case where the spectrum data having a wavelength bandwidth of 900 nm to 1100 nm is used for calibration, an independent component having a peak in one or both of the wavelengths of $940 \pm \alpha$ [nm] and $1025 \pm \alpha$ [nm] is selected from a plurality of independent components obtained by the independent component analysis shown in Fig. 1. Such independent component becomes a component waveform signal having a waveform close to those in three independent components IC1a to IC1c shown in Fig. 4A. The independent component (component waveform signal) is selected as follows.

Case 1

In a case where an independent component having a peak at $940 \pm \alpha$[nm] is present in the plurality of independent components obtained by the independent component analysis and an independent component having a peak at $1025 \pm \alpha$[nm] is not present therein, the independent component (close to the first independent component IC1a in (1) of Fig. 4A) having a peak at $940 \pm \alpha$[nm] is selected in Step T140.

Case 2

In a case where an independent component having a peak at $940 \pm \alpha$[nm] is not present in the plurality of independent components obtained by the independent component analysis and an independent component having a peak at $1025 \pm \alpha$[nm] is present therein, the independent component (close to the second independent component IC1b in (2) of Fig. 4A) having a peak at $1025 \pm \alpha$[nm] is selected in Step T140.

Case 3

In a case where an independent component having a peak at both of $940 \pm \alpha$[nm] and $1025 \pm \alpha$[nm] is present in the plurality of independent components obtained by the independent component analysis, the independent component (close to the third independent component IC1c in (3) of Fig. 4A) is selected in Step T140.

Further, in the case 3 described above, the correlation between the inner product value P and the component content C (that is, glucose concentration) in the graph shown in Fig. 1 (F) related to each of the three independent components IC1a to IC1c shown in Fig. 4A is acquired, and one independent component with the maximum correlation may be selected. In this manner, it is possible to improve the calibration precision.

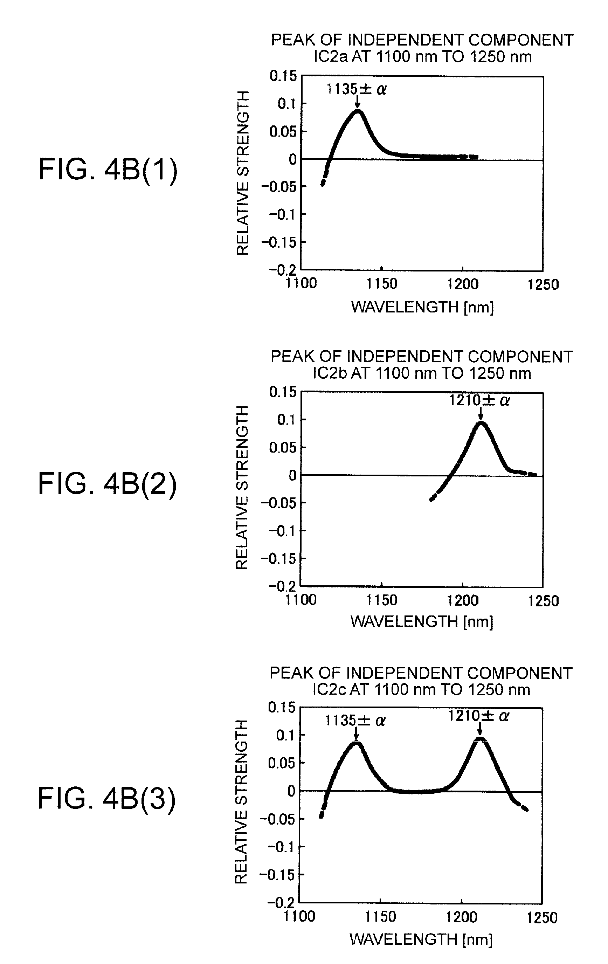

Fig. 4B shows an example of the preferable independent component in a case where spectrum data having a wavelength bandwidth of 1100 nm to 1250 nm is used for calibration. A first independent component IC2a shown in (1) of Fig. 4B is an independent component which has a peak at $1135 \pm \alpha$ [nm] and does not have a peak at $1210 \pm \alpha$ [nm]. A second independent component IC2b shown in (2) of Fig. 4B is an independent component which does not have a peak at $1135 \pm \alpha$ [nm] and has a peak at $1210 \pm \alpha$ [nm]. A third independent component IC2c shown in (3) of Fig. 4A is an independent component which has a peak at both of $1135 \pm \alpha$ [nm] and $1210 \pm \alpha$ [nm]. Similar to the case of Fig. 4A, as the value of $\alpha$, 30 is preferable and 20 is more preferable. Moreover, the third independent component IC2c corresponds to the sum of the first independent component IC2a and the second independent component IC2b.

In a case of using the spectrum data having a wavelength bandwidth of 1100 nm to 1250 nm for calibration, an independent component having a peak at one or both of $1135 \pm \alpha$ [nm] and $1210 \pm \alpha$ [nm] is selected from the plurality of independent components obtained by the independent component analysis shown in (A) to (F) in Fig. 1. Such an independent component has a waveform close to those in three independent components IC2a to IC2c shown in Fig. 4B. The independent component is selected in the same manner as in the case of using the wavelength bandwidth of 900 nm to 1100 nm.

Further, the selection of an independent component in Step T140 may be manually performed by an operator that operates the calibration curve generation device or may be automatically performed by the independent component selection unit in the calibration curve generation device.

Meanwhile, it is considered that a peak in the above-described wavelength is derived from light absorption of a CH group and a CH2 group included in glucose. In general, three overtone light absorption of the CH group and the CH2 group appears in a wavelength bandwidth of 900 nm to 1100 nm and two overtone light absorption thereof appears in a wavelength bandwidth of 1100 nm to 1250 nm. Further, the wavelength of two peaks appearing in the independent component having a wavelength bandwidth of 900 nm to 1100 nm with respect to a human body or blood is slightly shifted to the longer wavelength side than a single absorption peak of the CH group or the CH2 group due to the interaction with organism molecules. The reason why two peaks are individually handled is that the two peaks appear in independent components different from each other in some cases due to the influence of the absorption band of water. According to an experiment of the inventor of the present application, two peaks appear in independent components using spectrum data obtained by measuring a human body in many cases. Accordingly, in a case where a human body is used as a subject, it is preferable to use the independent component IClc (or IC2c) having two peaks. However, there is a possibility that the specific wavelength of the independent component depends on the site to be measured (for example, a hand or a foot) when the spectrum data is measured from a human body. Meanwhile, in a case where a glucose aqueous solution is used as a subject, peaks derived from the CH2 group are shifted to a shorter wavelength side and highly likely to become one peak.

When the selection of an independent component is finished, the inner product value P related to the plurality of samples is calculated using the selected independent component in Step T150 of Fig. 3 (see (D) and (E) in Fig. 1). Further, in a case where the inner product value P is calculated when the independent component is selected, the process of Step T150 can be omitted. In Step T160, a regression formula of a calibration curve is calculated from the relationship between the inner product value P related to the plurality of samples and the glucose concentration C measured in Step T120.

The single regression formula of the calibration curve is represented by the expression below.

$$C = u \cdot P + v \qquad \cdots (3)$$

Here, C represents glucose concentration, P represents an inner product value (mixing coefficient), and u and v represent an integer.

In addition, the independent component selected in Step T140 is used in the calibration process described in (A) to (D) in Fig. 2 and then calibration is performed.

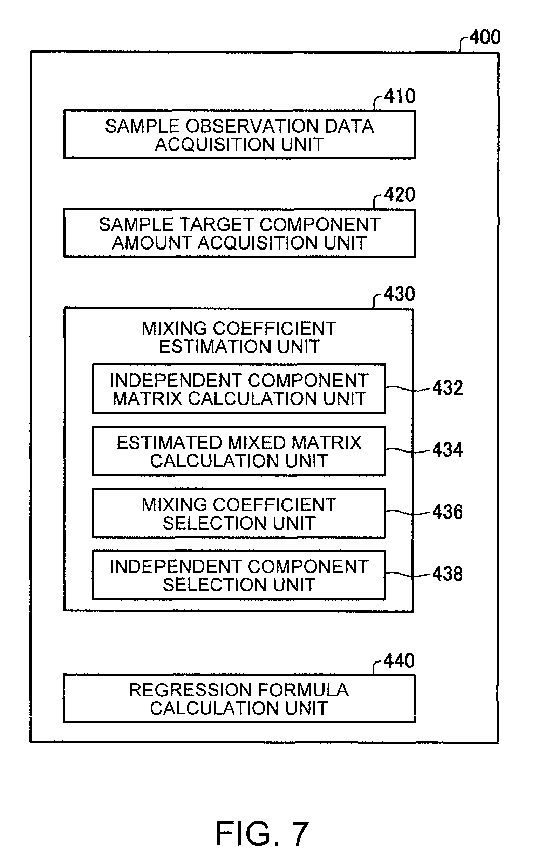

According to the experiment of the inventor of the present application, as a result of performing the calibration curve generation process using a human body as a subject and using the third independent component IClc shown in (3) of Fig. 4A, 7.2 mg/dL is obtained as calibration precision SEP (prediction standard deviation). In the calibration of noninvasive glucose concentration of the related art, the calibration precision SEP is approximately 20 mg/dL. Accordingly, in the present embodiment, calibration precision which is extremely higher than the noninvasive calibration of the related art can be achieved.

Further, in the present embodiment, since the characteristics of the independent components corresponding to glucose become clear, the calibration can be performed without collecting pieces of sample data for each subj ect. For example, the calibration of the glucose concentration of another test subject B can be performed using the independent component and the calibration curve determined using the measurement data of a test subject A.

Moreover, in a case where an independent component with a specific peak described above is not present in the plurality of independent components estimated in Step T130 of Fig. 3, it can be determined that measurement data sufficiently having the glucose information has not been acquired. Therefore, it is effective as an index for determining whether measurement conditions or algorithm parameters in the independent component analysis are appropriate. That is, in the case where an independent component with a specific peak described above is not present in the plurality of estimated independent components, it is preferable to perform the process in Fig. 3 again by changing a part of the measurement conditions or the algorithm parameters in the independent component analysis.

C. Calibration curve generation method:

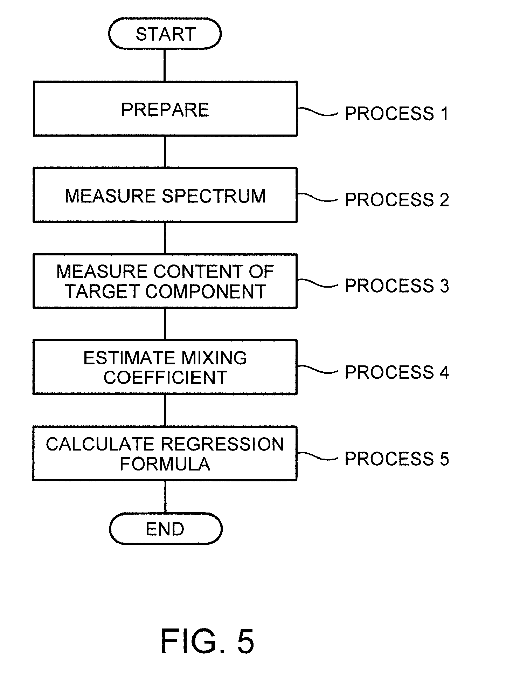

Fig. 5 is a flowchart showing a calibration curve generation method as an embodiment of the invention. The calibration curve generation method is configured of five processes 1 to 5. The respective processes 1 to 5 are performed in order. The respective processes 1 to 5 will be described in order. In addition, after this c section, a method which can be used for calibration of target components other than the blood sugar level described in Figs. 3, 4A, and 4B will be described unless otherwise noted.

Process 1

The process 1 is a preparation process and performed by an operator. The operator prepares the same kind of plural samples (for example, a glucose aqueous solution or a human body) in which the contents of target components are different from one other. In the example, n (n represents an integer of 2 or greater) samples are used. In a case of using a human body as a sample, the human body as the subject may be one or the same human body in different date and time can be used as plural samples.

Process 2

The process 2 is a process of measuring the spectrum and performed by the operator using a spectrometer. The operator measures the spectrum of spectral reflectance related to each of the samples by imaging each of the plurality of samples prepared in the process 1 using the spectrometer. The spectrometer is a known device in which light from an object to be measured passes through a spectroscope, the spectrum output from the spectroscope is received by an imaging surface of an imaging element, and thus the spectrum is measured. The spectrum of the spectral reflectance and the spectrum of the absorbance satisfy the relationship represented by the following expression.

$$[\text{Absorbance}] = -\log_{10}[\text{Reflectance}] \quad (4)$$

The spectrum of the measured spectral reflectance is transformed to the absorbance spectrum using the expression (4). The reason for conversion of the spectrum of the spectral reflectance to the absorbance spectrum is that a linear combination needs to be established in a mixed signal to be analyzed by the independent component analysis described below, and the linear combination related to the absorbance is established according to a Lambert-Beer's law. Therefore, in the process 2, the absorbance spectrum may be measured in place of the spectral reflectance spectrum. As the measurement results, absorbance distribution data showing characteristics with respect to the wavelength of the object to be measured is output. The absorbance distribution data is referred to as spectrum data.

Moreover, the spectral reflectance spectrum or the absorbance spectrum may be estimated from other measurement values instead of measuring these spectra using a spectroscope. For example, a sample is measured using a multi-band camera and the spectral reflectance or the absorbance spectrum may be estimated from an obtained multi-band image. As such an estimation method, a method described in JP-A-2001-99710 can be used.

Process 3

The process 3 is a process of measuring the content of target components and performed by the operator. The operator performs chemical analysis on each of the plurality of samples prepared in the process 1 and measures the content of the target components (for example, the amount of glucose) in regard to each of the samples. In a case where the content of the target components in the samples prepared in the process 1 is known, the process 3 can be omitted.

Process 4

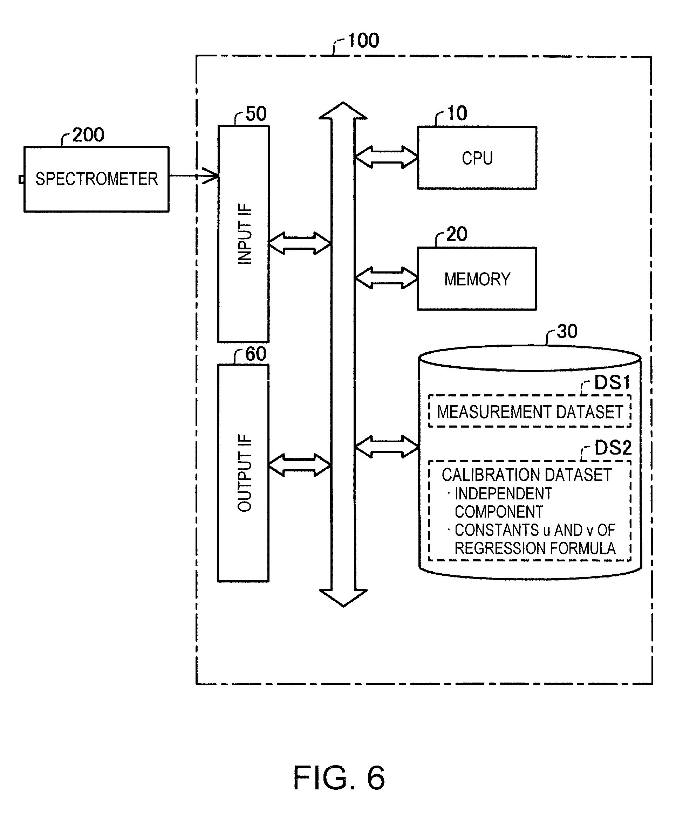

The process 4 is a process of estimating a mixing coefficient and typically performed using a computer. Fig. 6 is an explanatory diagram showing a computer 100 and peripheral devices thereof used in the process 4 and the process 5 described below. The computer 100 is electrically connected to a spectrometer 200.

The computer 100 is a known device including a CPU 10 that performs various processes or control by executing computer programs; a memory 20 (storage unit) which is a save location of data; a hard disk drive 30 that stores computer programs and data; an input I/F 50; and an output I/F 60.

Fig. 7 is a functional block diagram of a device used in the processes 4 and 5. A device 400 includes a sample observation data acquisition unit 410; a sample target component amount acquisition unit 420; a mixing coefficient estimation unit 430; and a regression formula calculation unit 440. The mixing coefficient estimation unit 430 includes an independent component matrix calculation unit 432; an estimated mixed matrix calculation unit 434; a mixing coefficient selection unit 436; and an independent component selection unit 438. Moreover, the sample observation data acquisition unit 410 and the sample target component amount acquisition unit 420 are implemented by, for example, the CPU 10 of Fig. 6 being in cooperation with the input I/F 50 and the memory 20. The mixing coefficient estimation unit 430, the independent component matrix calculation unit 432, the estimated mixed matrix calculation unit 434, the mixing coefficient selection unit 436, and the independent component selection unit 438 are implemented by, for example, the CPU 10 of Fig. 6 being in cooperation with the memory 20. Further, the regression formula calculation unit 440 is implemented by, for example, the CPU 10 of Fig. 6 being in cooperation with the memory 20. Moreover, these respective units can be implemented by other specific devices or a hardware circuit other than the computer shown in Fig. 6.

The independent component selection unit 438 has a function of performing a process of selecting an independent component described in Step T140 of Fig. 3. When the operator that operates the calibration curve generation device 400 inputs an instruction for selection using an input unit (not illustrated), the independent component selection unit 438 may be configured to perform selection of an independent component according to the input. Alternatively, the independent component selection unit 438 may automatically perform selection of an independent component without requiring the instruction of the operator. In addition, in a case where the selection of an independent component is performed by the procedures of Fig. 3, the estimated mixed matrix calculation unit 434 and the mixing coefficient selection unit 436 shown in Fig. 7 can be omitted. Hereinafter, the mixing coefficient estimation unit 430 is also simply referred to as an "estimation unit 430" and the sample target component amount acquisition unit 420 is also referred to as a "glucose concentration acquisition unit."

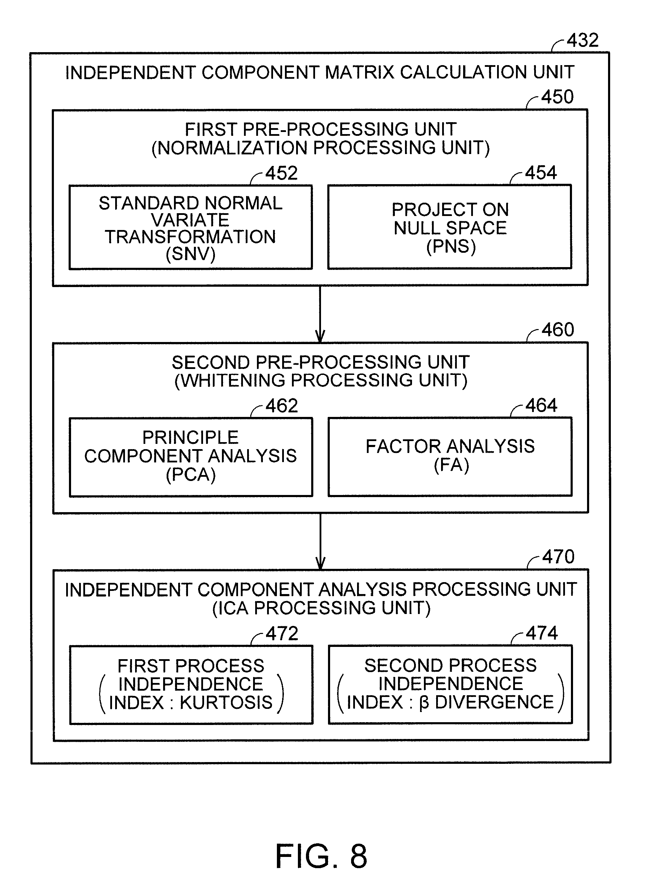

Fig. 8 is a functional block diagram showing an example of the internal configuration of the independent component matrix calculation unit 432. The independent component matrix calculation unit 432 includes a first pre-processing unit 450, a second pre-processing unit 460, and an independent component analysis processing unit 470. These three processing units 450, 460, and 470 acquire an independent component matrix (described below) by performing processing of data to be processed (the absorbance spectrum in the present embodiment) in this order. The processing contents of these respective units will be described below.

The spectrometer 200 shown in Fig. 6 is used in the process 2. The computer 100 acquires the absorbance spectrum obtained from the spectral distribution measured by the spectrometer 200 in the process 2 as spectrum data through the input I/F 50 (corresponding to the sample observation data acquisition unit 410 in Fig. 7). In addition, the computer 100 acquires the content of the target components measured in the process 3 through an operation of a keyboard performed by the operator (corresponding to the sample target component amount acquisition unit 420 in Fig. 7).

As a result of acquisition of the spectrum data and the content of target components described above, a dataset (hereinafter, referred to as a "measurement dataset") DS1 including the spectrum data and the content of target components is stored in the hard disk drive 30 of the computer 100.

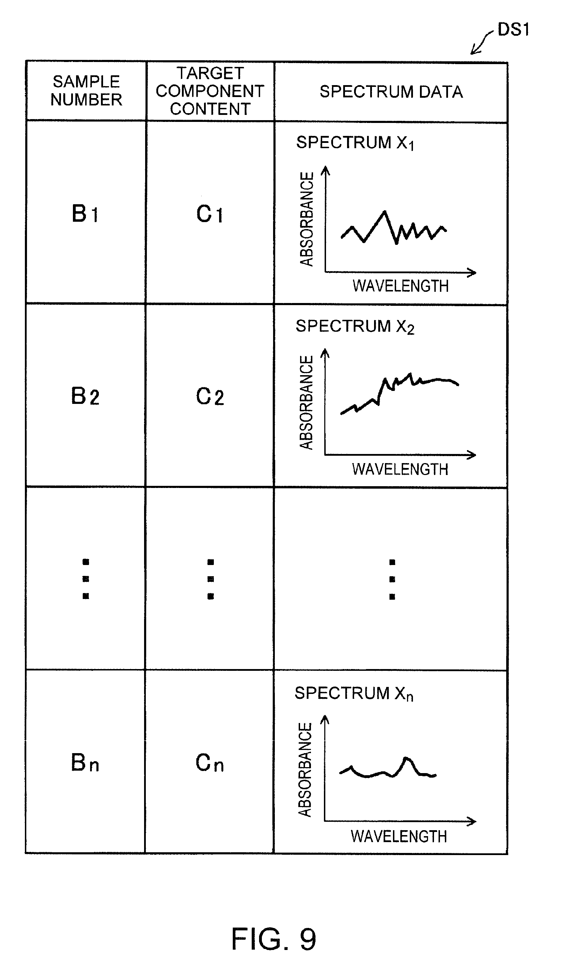

Fig. 9 is an explanatory diagram schematically showing the measurement dataset DS1 stored in the hard disk drive 30. As shown in the figure, the measurement dataset DS1 has a data structure including the sample numbers B1, B2, ... , Bn for identifying the plurality of samples prepared in the process 1, the target component contents C1, C2, ... , Cn related to each sample, and spectrum data X1, X2, ... , Xn related to each sample. In the measurement dataset DS1, the target component contents C1, C2, ... , Cn and the spectrum data X1, X2, ... , Xn are associated with the sample numbers B1, B2, ... , Bn so as to understand which sample the content and the data relate to.

The CPU 10 performs a process of estimating a mixing coefficient, which is the operation of the process 4, by loading a predetermined program stored in the hard disk drive 30 in the memory 20 and executing the program. Here, the predetermined program can be downloaded using a network such as the Internet from the outside. In the process 4, the CPU 10 functions as the mixing coefficient estimation unit 430 of Fig. 7.

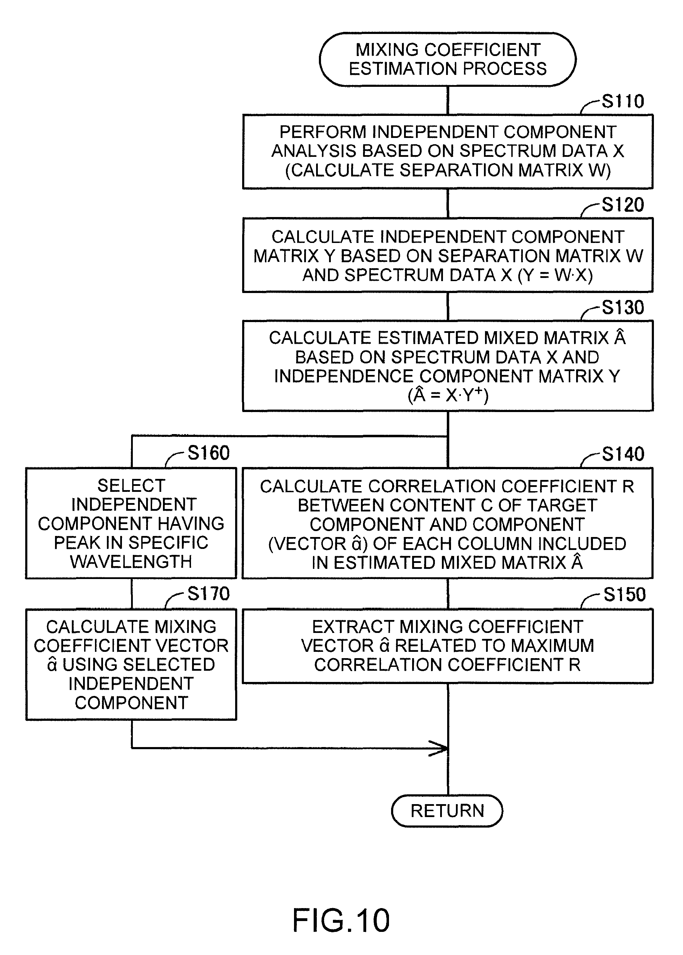

Fig. 10 is a flowchart showing the mixing coefficient estimation process performed by the CPU 10. When the process is started, the CPU 10 initially performs independent component analysis (Step S110).

The independent component analysis (ICA) is one of multi-dimensional signal analysis methods and is a technique of observing a mixed signal in which independent signals overlap each other under several conditions different from one another and separating independent original signals based on the observation. When the independent component analysis is used, the spectrum of the independent component can be estimated from the spectrum data (observation data) obtained in the process 2 by grasping the spectrum data obtained in the process 2 as data mixed with m independent components (unknown) including target components.

In the present embodiment, the independent component analysis is performed by the three processing units 450, 460, and 470 shown in Fig. 8 carrying out processes in this order. In the first pre-processing unit 450, pre-processing using one or both of standard normal variate transformation (SNV) 452 and project on null space (PNS) 454 can be performed. The SNV 452 is a process of obtaining normalized data in which the average value is 0 and the standard deviation is 1 by subtracting the average value of data to be processed and dividing with the standard deviation. The PNS 454 is a process for removing base line fluctuation included in the data to be processed. In the spectrum measurement, variation referred to as the base line fluctuation, for example, fluctuation in the average values of data for each piece of measurement data is generated between data due to various factors. Therefore, before the independent component analysis processing is performed, it is preferable to remove the factors of fluctuation. The PNS can be used as pre-processing capable of removing arbitrary base line fluctuation. Further, the PNS is described in, for example, "Extracting Chemical Information from Spectral Data with Multiplicative Light Scattering Effects by Optical Path-Length Estimation and Correction" written by Zeng-Ping Chen, Julian Morris, and Elaine Martin, 2006.

In addition, in a case where the SNV 452 is performed on the spectrum data obtained in the process 2 of Fig. 5, it is not necessary to perform a process using the PNS 454. Meanwhile, in a case where a process is performed using the PNS 454, it is preferable to perform any normalization process (for example, the SNV 452) thereafter.

Moreover, as the first pre-processing, a process other than the SNV and the PNS may be performed. Preferably, any normalization process is performed in the first pre-processing, but the normalization process may be omitted. Hereinafter, the first pre-processing unit 450 is referred to as the "normalization processing unit." The contents of these two processes 452 and 454 will be described below. Further, in a case where the data to be processed provided for the independent component matrix calculation unit 432 is normalized data, the first pre-processing may be omitted.

In the second pre-processing unit 460, pre-processing using any one of principal component analysis (PCA) 462 and factor analysis (FA) 464 can be performed. In addition, as the second pre-processing, processes other than the PCA or the FA may be performed. Hereinafter, the second pre-processing unit 460 is referred to as the "whitening processing unit." In a general technique of the ICA, dimension compression of data to be processed and decorrelation is performed as the second pre-processing. Since a transformation matrix to be acquired by the ICA is restricted to an orthogonal transformation matrix by the second pre-processing, the calculation amount of the ICA can be reduced. Such second pre-processing is referred to as "whitening," and the PCA is used in many cases. However, in a case where random noise is included in the data to be processed, an error may be generated in the results of the PCA due to the influence of the random noise. Here, in order to reduce the influence of the random noise, it is preferable to perform whitening using the FA having robustness with respect to noise in place of the PCA. The second pre-processing unit 460 of Fig. 8 can perform whitening by selecting any one of the PCA and the FA. The contents of the two processes 462 and 464 will be described below. Further, the whitening process may be omitted.

The independent component analysis processing unit (ICAprocessing unit) 470 estimates the spectrum of independent components by performing the ICA with respect to the spectrum data to which the first pre-processing and the second pre-processing are applied. The ICA processing unit 470 can perform analysis using any one of a first processing 472 using a kurtosis as an independence index and a second process 474 using $\beta$ divergence as the independence index. As the index for separating independent components from each other, the ICA uses higher order statistics representing independence of separated data as the independence index. The kurtosis is a typical independence index. However, in a case where an outlier such as spike noise is present in the data to be processed, the statistics including the outlier are calculated as the independence index. For this reason, an error is generated between original statistics related to the data to be processed and calculated statistics and this causes degradation of separation precision. Here, in order to reduce the influence from the outlier in the data to be processed, it is preferable to use an independence index which is unlikely to be affected by the outlier. It is possible to use the P divergence as the independence index having such characteristics. The contents of the kurtosis and the $\beta$ divergence will be described below. Further, as the independence index of the ICA, an index other than the kurtosis and the $\beta$ divergence may be used.

The contents of a typical process of the independent component analysis will be described below. It is assumed that a spectrum S (hereinafter, also simply referred to as an "unknown component") of m unknown components (sources) is provided as a vector of the expression (5) below and n pieces of spectrum data X obtained in the process 2 is provided as a vector in the expression (6) below. Further, respective elements (S1, S2, ... , Sm) included in the expression (5) are vectors (spectra). That is, the element S1 is represented by the expression (7). Elements (X1, X2, ... , Xn) included in the expression (6) are vectors and, for example, the element Xj is represented by the expression (8). The suffix j of the element Xj represents the number of wavelength bandwidths measuring a spectrum. Moreover, an element number m of the spectrum S of an unknown component represents an integer of 1 or greater and is empirically or experimentally determined in advance according to the kind of sample.

$$S = [S_1, S_2, \cdots, S_m]^\top \qquad \cdots (5)$$

$$X = [X_1, X_2, \cdots, X_n]^\top \qquad \cdots (6)$$

$$S_1 = \{S_{11}, S_{12}, \cdots, S_{1l}\} \qquad \cdots (7)$$

$$X_1 = \{X_{11}, X_{12}, \cdots, X_{1l}\} \qquad \cdots (8)$$

The respective unknown components are statistically independent. The unknown component S and the spectrum data X satisfy a relationship of the following expression.

$$X = A \cdot S \qquad \cdots (9)$$

In the expression (9), A represents a mixed matrix and can be represented by the expression (10) below. Further, the character "A" here needs to be written in bold as shown in the expression (10), but a normal character is used here because of the restriction of characters used in the specification. Hereinafter, similarly, other bold characters indicating matrixes are written in normal characters.

$$A = \begin{pmatrix} a_{11} & \cdots & a_{1m} \\ \vdots & \ddots & \vdots \\ a_{n1} & \cdots & a_{nm} \end{pmatrix} \quad \cdots (10)$$

A mixing coefficient aij included in the mixed matrix A indicates a degree of contribution of an unknown component Sj (j = 1 to m) to spectrum data Xi (i = 1 to n) which is observation data.

In a case where the mixed matrix A is known, a least square solution of the unknown component S can be simply acquired as A+·X using a pseudo inverse matrix A+ of A. However, in the present embodiment, since the mixed matrix A is unknown, the unknown component S and the mixed matrix A need to be estimated from only the observation data X. That is, as represented by the expression (11) below, a matrix (hereinafter, referred to as an "independent component matrix") Y indicating a spectrum of independent components is calculated using a separation matrix W of m x n from only the observation data X. As an algorithm acquiring the separation matrix W in the expression (11) below, various ones such as Infomax, fast independent component analysis (FastICA), and joint approximate diagonalization of eigenmatrices (JADE) can be employed.

$$Y = W \cdot X \quad \cdots (11)$$

The independent component matrix Y corresponds to an estimated value of the unknown component S. Accordingly, the expression (12) below can be obtained and the expression (13) below can be obtained by transforming the expression (12).

$$X = \hat{A} \cdot Y \quad \cdots (12)$$

$$\hat{A} = X \cdot Y^{+} \quad \cdots (13)$$

In the expression, ^A represents an estimated mixed matrix of A and Y+ indicates a pseudo inverse matrix of Y.

The estimated mixed matrix ^A (this notation is made because of the restriction of characters used in the specification and actually means the character with a symbol on the left side of the expression (13), and the same applies to other characters) obtained by the expression (13) can be represented by the following expression.

$$\hat{A} = \begin{pmatrix} \hat{a}_{11} & \cdots & \hat{a}_{1m} \\ \vdots & \ddots & \vdots \\ \hat{a}_{n1} & \cdots & \hat{a}_{nm} \end{pmatrix} \quad \cdots (14)$$

In Step S110 of Fig. 10, the CPU 10 performs a process of acquiring the separation matrix W described above. Specifically, the spectrum data X for each of the samples obtained in the process 2 and stored in the hard disk drive 30 in advance is set as an input, and the separation matrix W is acquired using any algorithm from among Infomax, FastICA, and JADE described above based on the input. Further, as shown in Fig. 8 described above, it is preferable that the first pre-processing unit 450 performs a normalization process and the second pre-processing unit 460 performs a whitening process as the pre-processing of the independent component analysis.

After the process of Step S110 is finished, the CPU 10 performs a process of calculating the independent component matrix Y based on the separation matrix W and the spectrum data X for each sample obtained in the process 2 and stored in the hard disk drive 30 in advance (Step S120). The calculation process is a process of performing calculation according to the expression (11) above. In the processes of Steps S110 and 120, the CPU 10 functions as the independent component matrix calculation unit 432 of Fig. 7.

Next, the CPU 10 performs a process of calculating the estimated mixed matrix ^A based on the spectrum data X for each sample stored in the hard disk drive 30 in advance and the independent component matrix Y calculated in Step S120 (Step S130). The calculation process is a process of performing calculation according to the expression (13) above.

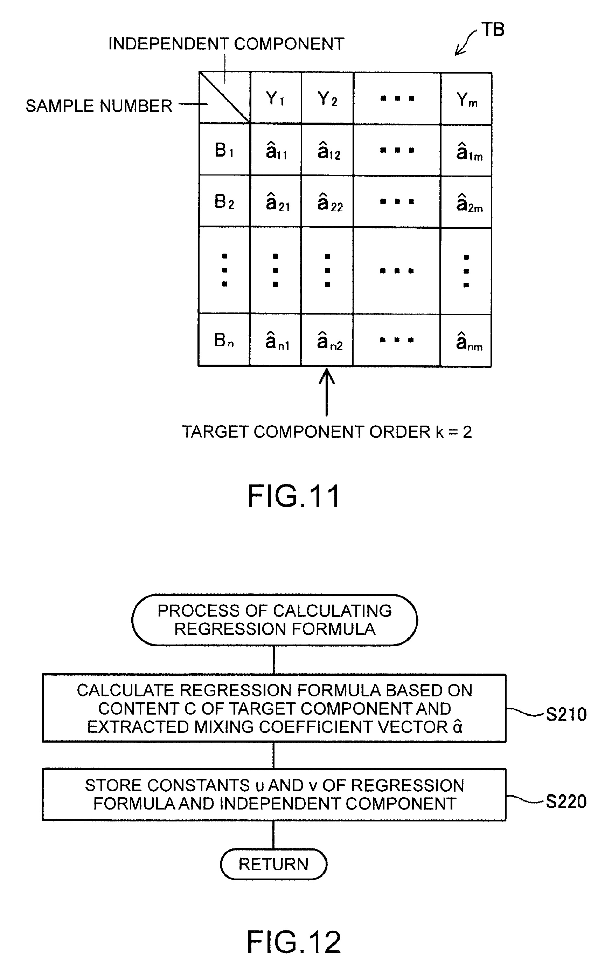

Fig. 11 is an explanatory diagram for describing the estimated mixed matrix ^A. In a table TB, each of the sample numbers B1, B2, ... , Bn is arranged in the vertical direction and each of the elements Y1, Y2, ... , Ym of the independent component matrix Y (hereinafter, referred to as an "independent component element") is arranged in the horizontal direction. Elements in the table TB determined from the sample number Bi (i = 1 to n) and the independent component element Yj (j = 1 to m) are the same as the elements ^aij (see the expression (14)) of the estimated mixed matrix ^A. From the table TB, it is understood that the elements ^aij of the estimated mixed matrix ^A represent the ratios of the respective independent component elements Y1, Y2, ... , Ym of samples. A target component order k shown in Fig. 11 will be described below. In the process of Step S130, the CPU 10 functions as the estimated mixed matrix calculation unit 434 of Fig. 7.

The estimated mixed matrix ^A is obtained by the processes up to Step S130. That is, the element (estimated mixing coefficient) ^aij of the estimated mixed matrix ^A is obtained. The estimated mixing coefficient ^aij corresponds to the inner product value P calculated in (D) to (F) in Fig. 1 in the example of (A) to (F) in Fig. 1. Subsequently, the process proceeds to Step S160 or S140.

In Step S160, the independent components are selected in accordance with the method explained in Step T140 in Fig. 3. The selection is performed by the independent component selection unit 438 in Fig. 7. In Step S170, the inner product values p in regard to the plurality of samples are calculated using the selected independent components and a mixing coefficient vector ^α whose elements are the inner product values p is obtained. The mixing coefficient vector ^α corresponds to mixing coefficients in one column shown in Fig. 11. Thus, the process of Fig. 10 terminates. When the selection of the independent components in Step S160 is not performed, the independent components are selected in Steps S140 and S150 described below. It is preferred that the calibration curve generation device 400 (Fig. 7) is configured such that the operator can arbitrarily select which one of the processes of Steps S160 and S170 and the processes of Step S140 and S150 is performed.

In Step S140, the CPU 10 acquires a correlation (degree of similarity) between the target component contents C1, C2, ... , Cn measured by the process 3 and components of respective columns included in the estimated mixed matrix ^A (hereinafter, referred to as the mixing coefficient vector ^α) calculated in Step S130. Specifically, a correlation between the target component content C (C1, C2, ... , Cn) and the mixing coefficient vector ^α1 in the first column (^a11, ^a21, ... , ^an1) is acquired, a correlation between the target component content C (C1, C2, ... , Cn) and the mixing coefficient vector ^α2 in the second column (^a12, ^a22, ... , ^an2) is acquired, and then a correlation with respect to the target component content C related to each column is sequentially acquired.

As the index indicating the degree of the correlation, a correlation coefficient R following the expression below can be used. The correlation coefficient R is referred to as Pearson's product-moment correlation coefficient.

$$R = \frac{\sum_{i=1}^{n}(C_i - \overline{C})(\hat{a}_{ik} - \overline{\hat{\alpha}_k})}{\sqrt{\sum_{i=1}^{n}(C_i - \overline{C})^2}\sqrt{\sum_{i=1}^{n}(\hat{a}_{ik} - \overline{\hat{\alpha}_k})^2}} \qquad \cdots (15)$$

-C and -^$\alpha_k$ each independently represent a target component amount and an average value of elements of a vector A ^$\alpha_k$.

As a result of Step S140 in Fig. 10, correlation coefficients Rj (j = 1, 2, ... , m) for each independent component (independent component spectrum) Yj are obtained. Subsequently, the CPU 10 specifies a coefficient with the maximum correlation, that is, a coefficient whose correlation is close to 1 from among the correlation coefficients Rj obtained in Step S140. Next, a column vector ^α obtained by the maximum correlation coefficient R is selected from the estimated mixed matrix ^A (Step S150).

The selection in Step S150 is to select one column from among a plurality of columns when considered with the table TB of Fig. 11. Elements of the selected column are mixing coefficients of independent components corresponding to target components. As a result of the selection, mixing coefficient vectors ^αk (^α1k, ^α2k, ... , ^αnk) are obtained. Here, k represents an integer of 1 to m. Further, the value of k may be temporarily stored in the memory 20 as a target component order indicating that which number of the independent component corresponds to the target component. The elements ^α1k, ^α2k, ... , ^αnk included in the mixing coefficient vectors ^αk correspond to "the mixing coefficient corresponding to the target component." In addition, in the example of Fig. 11, the "target component order k = 2" indicates "mixing coefficient vectors ^α2 = (^α12, ^α22, ... , ^αn2)" corresponding to an independent component Y2. Further, in the present specification, the term "order" means a "value indicating the position in a matrix." In the processes of Step S140 and S150, the CPU 10 functions as the estimated coefficient selection unit 436 of Fig. 7. After the process of Step S150 is performed, the CPU finishes the process of calculating the mixing coefficient. As a result, the process 4 is completed and then the process proceeds to the process 5.

Process 5

The process 5 is a process of calculating a regression formula and performed using the computer 100 in the same manner as in the process 4. In the process 5, the computer 100 performs the process of calculating a regression formula of a calibration curve. Moreover, the process 5 may be performed after the data up to the process 4 is moved to another computer or a device.

Fig. 12 is a flowchart showing the process of calculating a regression formula performed by the CPU 10 of the computer 100. When the process is started, the CPU 10 calculates a regression formula based on the target component contents C (C1, C2, ... , Cn) measured in the process 3 and the mixing coefficient vectors $^\wedge\alpha k$ ($^\wedge\alpha 1k$, $^\wedge\alpha 2k$, ... , $^\wedge\alpha nk$) obtained in Step S150 (or Step S170) (Step S210). The regression formula can be represented by the expression (16) below. In Step S210, constants u and v in the expression (16) are acquired.

$$C = u \cdot P + v \qquad \cdots (16)$$

Here, C represents a target component content, P represents an inner product between measurement data and an independent component, and u and v represent constants.

After the process in Step S210 is performed, the CPU 10 stores the constants u and v of the regression formula acquired in Step S210 and an independent component Yk (or an independent component selected in Step S160) corresponding to the target component order k (Fig. 11) determined in Step S150 in the hard disk drive 30 as a dataset DS2 for calibration (Step S220). Next, the CPU 10 exits to "return" and the process of calculating the regression formula is temporarily finished. As a result, the regression formula of a calibration curve can be acquired and the calibration curve generation method shown in Fig. 5 is finished. In the processes of Steps S210 and S220, the CPU 10 functions as the regression formula calculation unit 440 of Fig. 7.

D. Calibration method of target component:

The calibration method of a target component will be described below. The subject is set to be configured of the same components as those of a sample used when a calibration curve is generated. Specifically, the calibration method of a target component is performed using a computer. Moreover, the computer here may be the computer 100 used when a calibration curve is generated or another computer.

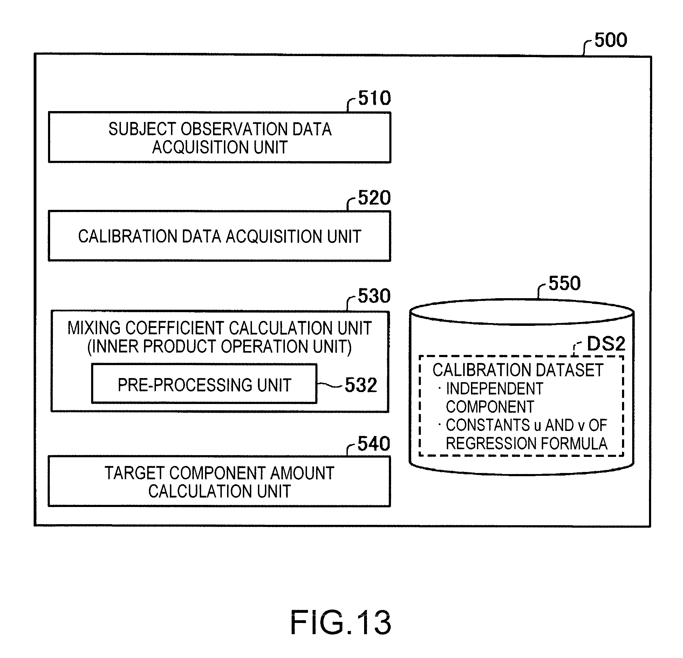

Fig. 13 is a functional block diagram of a device used when calibration is performed on a target component. A device 500 includes a subject observation data acquisition unit 510, a calibration data acquisition unit 520, a mixing coefficient calculation unit 530, a target component amount calculation unit 540, and a non-volatile storage device 550. The mixing coefficient calculation unit 530 includes a pre-processing unit 532. The pre-processing unit 532 has functions of both of the first pre-processing unit 450 and the second pre-processing unit 460 of Fig. 8. Since the mixing coefficient calculation unit 530 has a function of performing an inner product operation described in (A) to (C) in Fig. 2, the mixing coefficient calculation unit can be referred to as an "inner product operation unit." The subject observation data acquisition unit 510 is implemented by, for example, the CPU 10 of Fig. 6 being in cooperation with the input I/F 50 and the memory 20. The calibration data acquisition unit 520 is implemented by, for example, the CPU 10 of Fig. 6 being in cooperation with the memory 20 and the hard disk drive 30. The mixing coefficient calculation unit 530 and the target component amount calculation unit 540 are implemented by, for example, the CPU 10 of Fig. 6 being in cooperation with the memory 20. The calibration dataset DS2 (independent components and constants u and v of a regression formula) is stored in the non-volatile storage device 550. Further, the device in Fig. 13 may be mounted as another device or an electronic device which is different from the computer of Fig. 6. In this case, it is preferable that the device of Fig. 13 or the electronic device including the device includes a spectrometer.

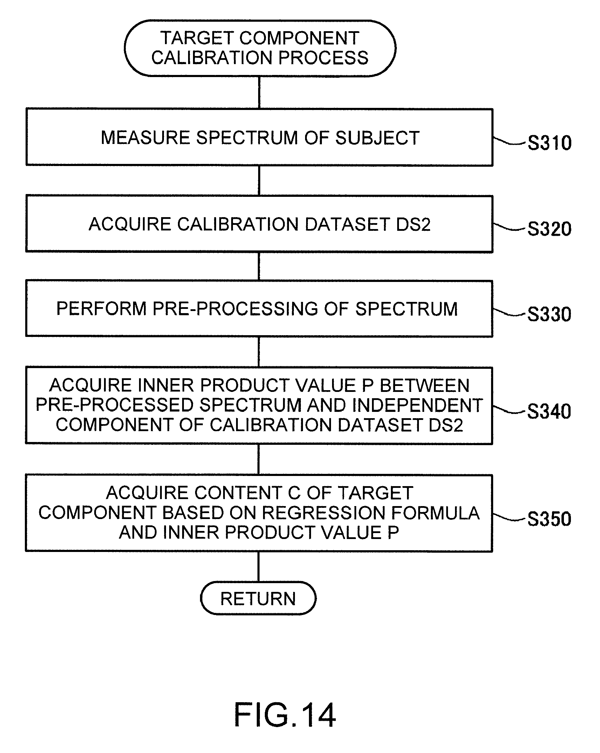

Fig. 14 is a flowchart showing the target component calibration process performed by the CPU 10 of the computer 100. The target component calibration process is implemented by the CPU 10 downloading a predetermined program, which is stored in the hard disk drive 30, in the memory 20 and executing the program. First, the CPU 10 performs a process of imaging the subject using a spectrometer (Step S310). The imaging of the subject in Step S310 can be performed in the same manner as in the process 2 and, as a result, an absorbance spectrum Xp of the subject is obtained. It is preferable that the spectrometer used in the calibration process is the same kind of device as that used for generation of a calibration curve for the purpose of suppressing errors. In order to further suppress errors, it is more preferable that the spectrometer used in the calibration process is the same as that used for generation of a calibration curve. In addition, similar to the process 2 of Fig. 5, instead of measuring the spectral reflectance spectrum or the absorbance spectrum with a spectroscope, these spectra may be estimated from other measurement values. The spectrum Xp of the absorbance of a subject obtained when the subject is imaged once is represented by a vector as in the expression below.

$$X_p = \{X_{p1}, X_{p2}, \cdots, X_{pl}\} \quad \cdots (17)$$

In the process of Step S310, the CPU 10 functions as the subject observation data acquisition unit 510 of Fig. 13. Next, the CPU 10 acquires the calibration dataset DS2 from the hard disk drive 30 (the non-volatile storage device 550 of Fig. 13) and stores the acquired calibration dataset DS2 in the memory 20 (Step S320). In the process of Step S320, the CPU 10 functions as the calibration data acquisition unit 520 of Fig. 13.

After the process of Step S320 is performed, pre-processing is performed with respect to the observation data (absorbance spectrum Xp) of the subject obtained in Step S310 (Step S330). As the pre-processing, it is preferable to perform the same processing as that (that is, the normalization process performed by the first pre-processing unit 450 and the whitening process performed by the second pre-processing unit 460) used in the process 4 (more specifically, Step S110 of Fig. 10) of Fig. 5 at the time when a calibration curve is generated.

Thereafter, the CPU 10 acquires the inner product value P between the independent component included in the calibration dataset DS2 and the pre-processed spectrum (pre-processed observation data) obtained in Step S330 (Step S340). The process of Step S340 corresponds to the process of (B) and (C) in Fig. 2 described above. Further, the inner product value P corresponds to the mixing coefficient calculated in Step S130 of Fig. 10 at the time when a calibration curve is generated. Therefore, the inner product value P is also referred to as a "mixing coefficient."

In the processes of Steps S330 and 340, the CPU 10 functions as the mixing coefficient calculation unit 530 of Fig. 13.

Next, the CPU 10 acquires the content C of the target component by reading the constants u and v of the regression formula included in the calibration dataset DS2 from the hard disk drive 30 (the non-volatile storage device 550 in Fig. 13) and substituting the constants u and v and the inner product value P obtained in Step S340 for the right side of the expression (16) above (Step S350). At this time, the constants u and v may be adjusted if necessary. The content C can be acquired as the unit volume or the mass of the target component per unit mass (for example, per 1 dL or per 100 g) of the subject. In the process of Step S350, the CUP 10 functions as the target component amount calculation unit 540 of Fig. 13. Next, the CPU 10 exits to "return" and finishes the target component calibration process.

Further, in the present embodiment, the content C acquired in Step S350 is set as the content of the target component of the subject, but, alternatively, the content C acquired in step S350 is corrected by a normalization coefficient used for normalization in Step S330 and then the corrected value may be used as the content to be acquired. Specifically, an absolute value (gram) of the content may be acquired by multiplying the content C by a standard deviation. According to the configuration, depending on the kind of target component, it is possible to make the content C have improved precision.

According to the above-described calibration method, the content of the target component can be acquired with high precision from one spectrum which is a measured value of the subject.

E. Various algorithms and influence:

Hereinafter, various algorithms used in the first pre-processing unit 450, the second pre-processing unit 460, and the independent component analysis processing unit 470 shown in Fig. 8 will be sequentially described.

E-1. First pre-processing (normalization process using SNV and PNS):

As the first pre-processing performed by the first pre-processing unit 450, standard normal variate transformation (SNV), and project on null space (PNS) can be used.

The SNV is given by the expression below.

$$z = \frac{x - x_{ave}}{\sigma} \quad \cdots (18)$$

Here, z represents processed data, x represents data to be processed (in the present example, the absorbance spectrum), xave represents an average value of data x to be processed, and σ represents a standard deviation of data x to be processed. As a result of the standard normal variate transformation, normalized data z in which the average value is 0 and the standard deviation is 1 is obtained.

When the PNS is performed, it is possible to decrease the base line fluctuation included in the data to be processed. In measurement of the data to be processed (the absorbance spectrum in the present embodiment), variation referred to as the base line fluctuation, for example, fluctuation in the average values of data for each piece of measurement data is generated between data due to various factors. Therefore, before the independent component analysis (ICA) is performed, it is preferable to remove the factors of fluctuation. The PNS can be used as pre-processing capable of decreasing the base line fluctuation in the data to be processed. Particularly, in regard to measurement data of an absorbance spectrum including an infrared region and a reflected light spectrum, since such base line fluctuation is large, application of the PNS is advantageous. Hereinafter, a principle in which the base line fluctuation included in data obtained by measurement (also simply referred to as "measurement data x") is removed by the PNS will be described. Further, as a typical example, a case where the measurement data is an absorbance spectrum including an infrared region or is a reflected light spectrum will be described. In this case, in regard to other kinds of measurement data (for example, audio data or the like), the PNS can be applied in the same manner as described above.

In an ideal system, the measurement data x (data x to be processed) is represented by the expression below using m (m is an integer of 2 or greater) independent components si (i = 1 to m) and respective mixing ratios ci.

$$x = \sum_{i-1}^{m} c_i s_i$$
$$= A \cdot s \qquad \cdots (19)$$

Here, A represents a matrix (mixed matrix) formed by a mixing ratio ci.

In the independent component analysis (ICA), the process is performed on the assumption of this model. However, various fluctuation factors (the state of a sample or a change in the measurement environment) are present in actual measurement data. For this reason, as a model in consideration of the fluctuation factors, a model expressing the measurement data x using the expression below can be considered.

$$x = b\sum_{i=1}^{m} c_i s_i + aE + b_1 f_1(\lambda) + b_2 f_2(\lambda) + \cdots b_g f_g(\lambda) + \varepsilon \qquad \cdots (20)$$

Here, the parameter b represents the amount of fluctuation in an amplitude direction of a spectrum; the parameter a represents the amount of constant base line fluctuation E (also referred to as "fluctuation in the average value"); the parameters b1, ..., bg represent the amount of g (g is an integer of 1 or greater) fluctuations f1(λ) to fg(λ) depending on the wavelength, and ε represents a fluctuation component other than those described above. Further, the constant base line fluctuation E is given by "E = {1, 1, 1, ... , 1}T (T on the right side represents transposition) and is a constant vector whose data length is equivalent to a data length N (the number of segments in the wavelength bandwidth) of the measurement data x. As a variable λ indicating a wavelength, N integers from 1 to N are used. That is, the variable λ corresponds to an ordinal number of the data length N (N is an integer of 2 or greater) of the measurement data x. At this time, fluctuations f1(λ), ... , fg(λ) depending on the wavelength are given by "f1(λ) = {f1(1), f1(2),..., f1(N)}T, ... , fg(λ) = {fg(1), fg(2), ... , fg(N)}T." Since these fluctuations are error factors in the ICA or calibration, it is desirable to remove the fluctuations in advance.

As the function f(λ), it is preferable to use one variable function in which the function f(λ) value monotonically increases according to an increase of λ in the range in which the value of λ is 1 to N. In the project on null space, the fluctuation included in the measurement data can be further reduced when a function other than an exponential function λα of λ in which an exponent α is an integer is used.

As a method of determining the functional types of a preferable function f(λ) and the number g thereof, experimental trial and errors may be employed or existing parameter estimation algorithms (for example, an expectation maximization method (EM) algorithm) may be used.

In the PNS, when a space formed of the above-described respective base line fluctuation component E and f1(λ) to fg(λ) is considered and the measurement data x is projected to a space (null space) without having these fluctuation components, data with reduced base line fluctuation component E and f1(λ) to fg(λ) can be obtained. As a specific operation, data z processed by the PNS is calculated by the expression below.

$$z = \left(1 - PP^+\right)x = b\sum_{i=1}^{m} c_i \mathbf{k}_i + \varepsilon^* \qquad \cdots \ (2\,1)$$

$$P = \left\{1, f_1(\lambda), f_2(\lambda) \cdots f_g(\lambda)\right\}$$

Here, P+ represents a pseudo inverse matrix of P. ki is obtained by projecting a constituent component si of the expression (20) to a null space without having fluctuation components. Further, $\varepsilon^*$ is obtained by projecting a fluctuation component $\varepsilon$ of the expression (20) to a null space.

Moreover, when normalization (for example, the SNV) is performed after the process of the PNS, it is possible to remove the influence of the fluctuation amount b in the amplitude direction of the spectrum in the expression (20).

When the ICA is performed on data pre-processed by the PNS, the obtained independent component becomes an estimated value of the component ki of the expression (21) and becomes a value different from the true constituent component si. However, since the mixing ratio ci is not changed from the original value in the expression (20), the mixing ratio ci does not affect the calibration process ((A) to (D) in Fig. 2 and Fig. 14) using the mixing ratio ci. In this manner, when the PNS is performed as the pre-processing of the ICA, since the true constituent component si cannot be obtained by the ICA, an idea in which the PNS is applied to the pre-processing of the ICA is not normally generated. Meanwhile, in the present embodiment, since the calibration process is not affected even when the PNS is performed as the pre-processing of the ICA, when the PNS is performed as the pre-processing, it is possible to perform calibration with high precision.

Further, the details of the PNS are described in "Extracting Chemical Information from Spectral Data with Multiplicative Light Scattering Effects by Optical Path-Length Estimation and Correction" written by Zeng-Ping Chen, Julian Morris, and Elaine Martin, 2006.

E-2. Second pre-processing (whitening process using PCA/FA)

As the second pre-processing performed by the second pre-processing unit 460, the principal component analysis (PCA) and the factor analysis (FA) can be used.

In a general technique of the ICA, dimension compression of data to be processed or decorrelation is performed as the pre-processing. Since a transformation matrix to be acquired by the ICA is restricted to an orthogonal transformation matrix by the pre-processing, the calculation amount of the ICA can be reduced. Such pre-processing is referred to as "whitening," and the PCA is used in many cases. The whitening using the PCA is described in Chapter 6 of "Independent Component Analysis" written by Aapo Hyvarinen, Juha Karhumen, and Erkki Oja, published by John Wiley & Sons, Inc., 2001 ("Independent Component Analysis," in February 2005, published by publishing department of Tokyo Denkki University).

However, in the PCA, in a case where random noise is included in the data to be processed, an error may be generated in the measurement results due to the influence of the random noise. Here, in order to reduce the influence of the random noise, it is preferable to perform whitening using the factor analysis (FA) having robustness with respect to noise in place of the PCA. Hereinafter, the principle of whitening using the FA will be described.

As described above, generally in the ICA, a linear mixed model (the expression (19) above) which represents the data x to be processed as a linear sum of the constituent components si is assumed and the mixing ratio ci and the constituent components si are acquired. However, in actual data, random noise other than the constituent components si is added in many cases. Here, as a model in consideration with the random noise, a model expressing the measurement data x using the expression below can be considered.

$$x = A \cdot s + \rho \qquad \cdots \ (2\,2)$$

Here, $\rho$ represents the random noise.

In addition, whitening in consideration with the noise mixed model is performed and then an estimate of the mixed matrix A and the independent component si can be obtained by performing the ICA.

In the FA of the present embodiment, it is assumed that the independent component si and the random noise $\rho$ respectively follow normal distribution $N(0, Im)$ and $N(0, \Sigma)$. Moreover, as is generally known, the first parameter x1 in normal distribution $N(x1, x2)$ represents an expected value and the second parameter x2 therein represents a standard deviation. At this time, since the data x to be processed becomes a linear sum of variables following the normal distribution, the data x to be processed also follows the normal distribution. Here, when a covariance matrix of the data x to be processed is set as V[x], the normal distribution followed by the data x to be processed can be implemented as N(0, V[x]). At this time a likelihood function related to the covariance matrix V[x] of the data x to be processed can be calculated by the following procedures.

First, when it is assumed that the independent components si are orthogonal to each other, the covariance matrix V [x] of the data x to be processed is calculated by the expression below.

$$V[\mathbf{x}] = E[\mathbf{x}\mathbf{x}^T] = \mathbf{A}\mathbf{A}^T + \Sigma \qquad \cdots (2\ 3)$$

Here, $\Sigma$ represents a covariance matrix of the noise $\rho$.

In this manner, the covariance matrix V[x] can be represented by the mixed matrix A and the covariance matrix $\Sigma$ of noise. At this time, a log-likelihood function L (A, $\Sigma$) is given by the expression below.

$$L(\mathbf{A},\Sigma) = -\frac{n}{2}\left\{ tr\left(\left(\mathbf{A}\mathbf{A}^T + \Sigma\right)^{-1}\mathbf{C}\right) + \log\left(\det\left(\mathbf{A}\mathbf{A}^T + \Sigma\right)\right) + m\log 2\pi \right\} \qquad \cdots (2\ 4)$$

Here, n represents the number of pieces of data x, m represents the number of independent components, an operator tr represents a trace of a matrix (sum of diagonal components), and an operator det represents a determinant. In addition, C represents a sample covariance matrix acquired by sample calculation from the data x and is calculated by the expression below.

$$\mathbf{C} = \frac{1}{n}\sum_{i=1}^{n}\mathbf{x}_i\mathbf{x}_i^T \qquad \cdots (2\ 5)$$

The covariance matrix $\Sigma$ of the mixed matrix A and the noise can be acquired by the maximum-likelihood method using the log-likelihood function L (A, $\Sigma$) of the expression (24). A matrix which is hardly affected by the random noise $\rho$ of the expression (22) is obtained as the mixed matrix A. This is the basic principle of the FA. Further, as the algorithm of the FA, there are various algorithms using an algorithm other than the maximum likelihood method. In the present embodiment, various kinds of FAs can be used.

The estimated value obtained by the FA is only a value of AAT and the influence of the random noise is reduced and the data can be decorrelated in a case where a mixed matrix A suitable for the value of AAT is determined, but a plurality of constituent components si cannot be uniquely determined because the degree of freedom of rotation remains. Meanwhile, the ICA is a process of reducing the degree of freedom of rotation of the plurality of constituent components si such that the plurality of constituent components si are orthogonal to each other. In the present embodiment, the values of the mixed matrix A acquired by the FA are used as a whitened matrix and arbitrary properties with respect to the remaining rotation are specified by the ICA. In this manner, after a robust whitening process is performed on the random noise, independent constituent components si which are orthogonal to each other can be determined by performing the ICA. In addition, as a result of such process, the calibration precision in regard to the constituent components si can be improved by reducing the influence of the random noise.

E-3. ICA (kurtosis and $\beta$ divergence as independence index):

In the independent component analysis (ICA), as the index for separating independent components from each other, higher order statistics representing independence of separated data are used as the independence index. The kurtosis is a typical independence index. The ICA using the kurtosis as the independence index is described in Chapter 8 of "Independent Component Analysis" written by Aapo Hyvarinen, Juha Karhumen, and Erkki Oja, published by John Wiley & Sons, Inc., 2001 ("Independent Component Analysis," in February 2005, published by publishing department of Tokyo Denkki University).

However, in a case where an outlier such as spike noise is present in the data to be processed, the statistics including the outlier are calculated as the independence index. For this reason, an error is generated between original statistics related to the data to be processed and calculated statistics and this causes degradation of separation precision.

Here, it is preferable to use an independence index which is unlikely to be affected by the outlier in the data to be processed. It is possible to use the β divergence as the independence index having such characteristics. Hereinafter, the principle of P divergence as the independence index of the ICA will be described.

As described above, generally in the ICA, a linear mixed model (the expression (19) above) which represents the data x to be processed as a linear sum of the constituent components si is assumed and the mixing ratio ci and the constituent components si are acquired. An estimated value y of the constituent component s acquired by the ICA is represented by "y = W·y" using a separation matrix W. It is desired that the separation matrix W is an inverse matrix of the mixed matrix A.

Here, a log-likelihood function L (^W) of an estimated value ^W of the separation matrix W can be represented by the expression below.

$$L\left(\hat{\mathbf{W}}\right) = \frac{1}{N}\sum_{t=1}^{N} l\left(\mathbf{x}(t), \hat{\mathbf{W}}\right) \quad \cdots \quad (2\,6)$$

Here, an element of an integration symbol $\sum$ is a log likelihood in each data point x(t). The log-likelihood function L (^W) can be used as an independence index in the ICA. The technique of P divergence is a method of transforming the log-likelihood function L (^W) with respect to an outlier such as spike noise in data for the purpose of suppressing the influence of the outlier by acting an appropriate function on the log-likelihood function L (^W).

In a case of using the β divergence as the independence index, first, the log-likelihood function L (^W) is transformed by the expression below using a function Φβ selected in advance.

$$L_{\Phi}\left(\hat{\mathbf{W}}\right) = \frac{1}{N}\sum_{t=1}^{N} \Phi_{\beta}\left(l\left(\mathbf{x}(t), \hat{\mathbf{W}}\right)\right) \quad \cdots \quad (2\,7)$$

In addition, the function LΦ (^W) is considered as a new likelihood function.

As the function Φβ for reducing the influence of an outlier such as spike noise, a function in which the value of the function Φβ is exponentially attenuated when the value of the log likelihood (value in parentheses of the function Φβ) becomes smaller is considered. As such a function Φβ, the following function can be used.

$$\Phi_{\beta}(z) = \frac{1}{\beta}\{\exp(\beta z) - 1\} \quad \cdots \quad (2\,8)$$

In this function, the function value with respect to each data point z (log likelihood in the expression (27) above) becomes smaller when the value of P becomes larger. The value of P can be empirically determined and can be set as, for example, approximately 0.1. Further, the function Φβ is not particularly limited to the function of the expression (28) and another function in which the function value with respect to each data point z becomes smaller when the value of β becomes larger can be used.

When such β divergence is used as the independence index, the influence of an outlier such as spike noise can be appropriately suppressed. When the likelihood function LΦ (^W) as in the expression (27) is considered, a pseudo distance between probability distribution to be minimized in correspondence with the maximization of likelihood is the P divergence. When the ICA using such β divergence as the independence index is performed, the influence of an outlier such as spike noise is reduced and the calibration precision related to the constituent component si can be improved.

In addition, the ICA using the P divergence is described in "Robust Blind Source Separation by β-Divergence" written by Minami Mihoko and Shinto Eguchi, 2002.

F. Modification Examples:

The invention is not limited to examples or modification examples and can be implemented with various aspects within the range not departing from the scope of the invention, and the following modifications are possible.

• Modification Example 1:

In the embodiment, the element number m of the spectrum S of an unknown component is empirically and experimentally determined in advance, but the element number m of the spectrum S of an unknown component may be determined based on the information criterion known as the minimum description length (MDL) or the akaike information criteria (AIC). In a case of using the MDL or the like, the element number m of the spectrum S of an unknown component can be automatically determined by an operation from observation data of samples. In addition, for example, the MDL is described in "Independent component analysis for noisy data? MEG data analysis, 2000."

• Modification Example 2:

In the embodiment, the subject as a target of the calibration process is configured of the sample components as those of a sample used when the calibration curve is generated, but unknown components other than the components which are the same as those of the sample used when the calibration curve is generated may be included in the subject. This is because the inner product between independent components is set as 0 and the inner product of independent components corresponding to the unknown component is considered as 0, the influence of the unknown component in a case of acquiring a mixing coefficient using the inner product can be ignored.

• Modification Example 3:

The computer used in the embodiment may be configured as a dedicated device. For example, the device shown in Fig. 7 or 13 may be implemented only by a hardware circuit. Alternatively, a part of the function of the device shown in Fig. 7 or 13 is implemented by the hardware circuit and another part may be implemented by software.

• Modification Example 4:

In the embodiment, an input of a spectrum of the spectral reflectance related to a sample or a subject is performed by inputting the spectrum measured by a spectrometer, but the invention is not limited thereto. For example, a spectrum is estimated from a plurality of band images whose wavelength bandwidths are different from each other and the spectrum may be input. The band images can be obtained by imaging a sample or a subject using a multi-band camera including a filter capable of changing a transmission wavelength bandwidth.

• Modification Example 5:

In the example described above, an independent component having a peak in the wavelength suitable for glucose when the independent component is selected using the independent component analysis is selected, but, alternatively, a component having a peak in the wavelength suitable for glucose may be selected when the main component is selected using a technique of main component analysis or PLS regression analysis. Even in this case, by performing the calibration curve generation process or the calibration process of the glucose concentration using the main component in place of the independent component, it is possible to improve calibration precision.
Further, elements other than the elements described in the aspects from among the constituent elements in each of the examples and modification examples described above are additional elements and can be appropriately omitted.

**Claims**

1. A calibration curve generation device which generates a calibration curve used for deriving glucose concentration of a subject from observation spectral data of the subject including glucose, the device comprising:

   a sample observation data acquisition unit that acquires the observation spectral data related to a plurality of samples of the subject;
   a glucose concentration acquisition unit that acquires the glucose concentration related to each of the samples;
   an estimation unit that estimates a plurality of independent components or main components when the observation spectral data for each of the samples is divided to the plurality of independent components or the main components; and
   a regression formula calculation unit that acquires a regression formula of the calibration curve based on the glucose concentrations of the plurality of samples and a mixing coefficient of the independent components or the main components in the observation spectral data for each of the samples,
wherein the estimation unit selects a component waveform signal having a peak in a wavelength selected in advance as the independent components or the main components.

2. The calibration curve generation device according to claim 1, wherein the wavelength selected in advance is one of (i) at least one wavelength between wavelengths of 940 $\pm$ 30 nm and 1025 $\pm$ 30 nm and (ii) at least one wavelength between wavelengths of 1135 $\pm$ 30 nm and 1210 $\pm$ 30 nm.

3. The calibration curve generation device according to claim 1, wherein the estimation unit includes an independent component matrix calculation unit that calculates an independent component matrix including the plurality of independent components and an independent component selection unit that selects an independent component having a peak in the wavelength selected in advance from the independent component matrix.

4. A target component calibration device which acquires glucose concentration related to a subject including glucose as a target component, the device comprising:

   a subject observation data acquisition unit that acquires observation spectral data related to the subject;
   a calibration data acquisition unit that acquires calibration data including an independent component or a main component corresponding to the glucose and a single regression formula for calibration;
   a mixing coefficient calculation unit that acquires a mixing coefficient with respect to the glucose related to the subject based on the observation spectral data and the calibration data related to the subject; and
   a target component amount calculation unit that calculates the glucose concentration based on the single regression formula indicating a relationship between the mixing coefficient and the glucose concentration corresponding to the glucose and the mixing coefficient acquired by the mixing coefficient calculation unit,
   wherein the mixing coefficient calculation unit uses a component having a peak in the wavelength selected in advance as the independent component or the main component with respect to the glucose.

5. The target component calibration device according to claim 4, wherein the wavelength selected in advance is one of (i) at least one wavelength between wavelengths of 940 $\pm$ 30 nm and 1025 $\pm$ 30 nm and (ii) at least one wavelength between wavelengths of 1135 $\pm$ 30 nm and 1210 $\pm$ 30 nm.

6. An electronic device comprising the target component calibration device according to claim 4.

7. An electronic device comprising the target component calibration device according to claim 5.

INDEPENDENT COMPONENT ANALYSIS (ICA)

INDEPENDENT
COMPONENT IC1

WAVEFORM AFTER
PRE-PROCESSING

PRE-
PROCESSING

**FIG. 1A**

**FIG. 1B**

INDEPENDENT
COMPONENT IC2

**FIG. 1C**

WAVEFORM AFTER
PRE-PROCESSING

INNER
PRODUCT
×

INDEPENDENT
COMPONENT IC1

INNER
PRODUCT
VALUE

CALIBRATION CURVE

C=uP+v

INNER PRODUCT
VALUE P

**FIG. 1D**

**FIG. 1E**

**FIG. 1F**

ABSORBANCE

1.0

0.7

0.4

900    1000    1100
WAVELENGTH [nm]

## FIG. 2A

PRE-
PROCESSING →

WAVEFORM AFTER
PRE-PROCESSING

ABSORBANCE

2.0

0.0

-1.5

900    1000    1100
WAVELENGTH [nm]

## FIG. 2B

INNER
PRODUCT

×

INDEPENDENT
COMPONENT IC1

0.04

0.00

-0.10

900    1000    1100
WAVELENGTH [nm]

## FIG. 2C

INNER
PRODUCT
VALUE

CALIBRATION CURVE

COMPONENT
CONTENT C

$C = uP + v$

C

INNER PRODUCT
VALUE P

## FIG. 2D

EP 3 035 206 A1

FIG. 3

PEAK OF INDEPENDENT COMPONENT
IC1a AT 900 nm TO 1100 nm

FIG. 4A(1)

PEAK OF INDEPENDENT COMPONENT
IC1b AT 900 nm TO 1100 nm

FIG. 4A(2)

PEAK OF INDEPENDENT COMPONENT
IC1c AT 900 nm TO 1100 nm

FIG. 4A(3)

PEAK OF INDEPENDENT COMPONENT
IC2a AT 1100 nm TO 1250 nm

FIG. 4B(1)

PEAK OF INDEPENDENT COMPONENT
IC2b AT 1100 nm TO 1250 nm

FIG. 4B(2)

PEAK OF INDEPENDENT COMPONENT
IC2c AT 1100 nm TO 1250 nm

FIG. 4B(3)

START

PREPARE ⟩— PROCESS 1

MEASURE SPECTRUM ⟩— PROCESS 2

MEASURE CONTENT OF
TARGET COMPONENT ⟩— PROCESS 3

ESTIMATE MIXING
COEFFICIENT ⟩— PROCESS 4

CALCULATE REGRESSION
FORMULA ⟩— PROCESS 5

END

FIG. 5

FIG. 6

400

410

SAMPLE OBSERVATION DATA
ACQUISITION UNIT

420

SAMPLE TARGET COMPONENT
AMOUNT ACQUISITION UNIT

430

MIXING COEFFICIENT
ESTIMATION UNIT

INDEPENDENT COMPONENT
MATRIX CALCULATION UNIT — 432

ESTIMATED MIXED MATRIX
CALCULATION UNIT — 434

MIXING COEFFICIENT
SELECTION UNIT — 436

INDEPENDENT COMPONENT
SELECTION UNIT — 438

440

REGRESSION FORMULA
CALCULATION UNIT

FIG. 7

432

INDEPENDENT COMPONENT MATRIX CALCULATION UNIT

450

FIRST PRE-PROCESSING UNIT
(NORMALIZATION PROCESSING UNIT)

452

STANDARD NORMAL
VARIATE
TRANSFORMATION
(SNV)

454

PROJECT ON
NULL SPACE
(PNS)

460

SECOND PRE-PROCESSING UNIT
(WHITENING PROCESSING UNIT)

462

PRINCIPLE
COMPONENT ANALYSIS
(PCA)

464

FACTOR ANALYSIS
(FA)

470

INDEPENDENT COMPONENT ANALYSIS PROCESSING UNIT
(ICA PROCESSING UNIT)

472

$\left(\begin{array}{c}\text{FIRST PROCESS}\\\text{INDEPENDENCE}\\\text{INDEX : KURTOSIS}\end{array}\right)$

474

$\left(\begin{array}{c}\text{SECOND PROCESS}\\\text{INDEPENDENCE}\\\text{INDEX : }\beta\text{ DIVERGENCE}\end{array}\right)$

# FIG. 8

DS1

| SAMPLE NUMBER | TARGET COMPONENT CONTENT | SPECTRUM DATA |
|---|---|---|
| $B_1$ | $C_1$ | SPECTRUM $X_1$ <br> ABSORBANCE / WAVELENGTH |
| $B_2$ | $C_2$ | SPECTRUM $X_2$ <br> ABSORBANCE / WAVELENGTH |
| ⋮ | ⋮ | ⋮ |
| $B_n$ | $C_n$ | SPECTRUM $X_n$ <br> ABSORBANCE / WAVELENGTH |

FIG. 9

```
        ╭─────────────────────────╮
        │   MIXING COEFFICIENT    │
        │   ESTIMATION PROCESS    │
        ╰─────────────────────────╯
                     │                      ⌐S110
        ┌─────────────────────────────────────┐
        │ PERFORM INDEPENDENT COMPONENT        │
        │ ANALYSIS BASED ON SPECTRUM DATA X    │
        │ (CALCULATE SEPARATION MATRIX W)      │
        └─────────────────────────────────────┘
                     │                      ⌐S120
        ┌─────────────────────────────────────┐
        │ CALCULATE INDEPENDENT COMPONENT      │
        │ MATRIX Y BASED ON SEPARATION MATRIX W│
        │ AND SPECTRUM DATA X (Y = W·X)        │
        └─────────────────────────────────────┘
                     │                      ⌐S130
        ┌─────────────────────────────────────┐
        │ CALCULATE ESTIMATED MIXED MATRIX Â   │
        │ BASED ON SPECTRUM DATA X AND         │
        │ INDEPENDENCE COMPONENT MATRIX Y      │
        │ (Â = X·Y⁺)                           │
        └─────────────────────────────────────┘
```

S160

SELECT INDEPENDENT COMPONENT HAVING PEAK IN SPECIFIC WAVELENGTH

S140

CALCULATE CORRELATION COEFFICIENT R BETWEEN CONTENT C OF TARGET COMPONENT AND COMPONENT (VECTOR â) OF EACH COLUMN INCLUDED IN ESTIMATED MIXED MATRIX Â

S170

CALCULATE MIXING COEFFICIENT VECTOR â USING SELECTED INDEPENDENT COMPONENT

S150

EXTRACT MIXING COEFFICIENT VECTOR â RELATED TO MAXIMUM CORRELATION COEFFICIENT R

```
        ╭─────────────╮
        │   RETURN    │
        ╰─────────────╯
```

# FIG.10

INDEPENDENT COMPONENT

SAMPLE NUMBER

| | $Y_1$ | $Y_2$ | $\cdots$ | $Y_m$ |
|---|---|---|---|---|
| $B_1$ | $\hat{a}_{11}$ | $\hat{a}_{12}$ | $\cdots$ | $\hat{a}_{1m}$ |
| $B_2$ | $\hat{a}_{21}$ | $\hat{a}_{22}$ | $\cdots$ | $\hat{a}_{2m}$ |
| $\vdots$ | $\vdots$ | $\vdots$ | $\cdots$ | $\vdots$ |
| $B_n$ | $\hat{a}_{n1}$ | $\hat{a}_{n2}$ | $\cdots$ | $\hat{a}_{nm}$ |

TB

TARGET COMPONENT ORDER k = 2

FIG.11

PROCESS OF CALCULATING
REGRESSION FORMULA

CALCULATE REGRESSION FORMULA BASED ON
CONTENT C OF TARGET COMPONENT AND
EXTRACTED MIXING COEFFICIENT VECTOR â     ~S210

STORE CONSTANTS u AND v OF REGRESSION
FORMULA AND INDEPENDENT COMPONENT     ~S220

RETURN

FIG.12

500

510

SUBJECT OBSERVATION DATA
ACQUISITION UNIT

520

CALIBRATION DATA ACQUISITION UNIT

550

530

MIXING COEFFICIENT CALCULATION UNIT
(INNER PRODUCT OPERATION UNIT)

PRE-PROCESSING UNIT — 532

DS2

CALIBRATION DATASET
· INDEPENDENT
COMPONENT
· CONSTANTS u AND v OF
REGRESSION FORMULA

540

TARGET COMPONENT AMOUNT
CALCULATION UNIT

FIG.13

```
      ┌─────────────────────────┐
      │    TARGET COMPONENT     │
      │   CALIBRATION PROCESS   │
      └─────────────────────────┘
                   │
   ┌───────────────────────────────────┐
   │   MEASURE SPECTRUM OF SUBJECT     │ ── S310
   └───────────────────────────────────┘
                   │
   ┌───────────────────────────────────┐
   │   ACQUIRE CALIBRATION DATASET DS2 │ ── S320
   └───────────────────────────────────┘
                   │
   ┌───────────────────────────────────┐
   │  PERFORM PRE-PROCESSING OF SPECTRUM│ ── S330
   └───────────────────────────────────┘
                   │
   ┌───────────────────────────────────┐
   │ ACQUIRE INNER PRODUCT VALUE P BETWEEN │
   │ PRE-PROCESSED SPECTRUM AND INDEPENDENT│ ── S340
   │ COMPONENT OF CALIBRATION DATASET DS2  │
   └───────────────────────────────────┘
                   │
   ┌───────────────────────────────────┐
   │    ACQUIRE CONTENT C OF TARGET    │
   │ COMPONENT BASED ON REGRESSION FORMULA│ ── S350
   │    AND INNER PRODUCT VALUE P      │
   └───────────────────────────────────┘
                   │
              ┌──────────┐
              │  RETURN  │
              └──────────┘
```

# FIG.14

Europäisches
Patentamt

European
Patent Office

Office européen
des brevets

# EUROPEAN SEARCH REPORT

Application Number

EP 15 18 8542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 784 484 A1 (SEIKO EPSON CORP [JP]) 1 October 2014 (2014-10-01) | 1,3,4,6,7 | INV. G06F17/18 A61B5/145 A61B5/1455 A61B5/1495 |
| A | * claims 1,12 * * paragraphs [0057], [0168] * * paragraph [0127] * * paragraphs [0163] - [0167] * ----- | 2,5 | |
| A | HAHN S ET AL: "IDENTIFICATION OF PURE COMPONENT SPECTRA BY INDEPENDENT COMPONENT ANALYSIS IN GLUCOSE PREDICTION BASED ON MID-INFRARED SPECTROSCOPY", APPLIED OPTICS, vol. 45, no. 32, 10 November 2006 (2006-11-10), pages 8374-8380, XP001501885, ISSN: 0003-6935, DOI: 10.1364/AO.45.008374 * section 1 * * section 3.A and figure 2 * ----- | 1-7 | |
| A | MARUO K ET AL: "New methodology to obtain a calibration model for noninvasive near-infrared blood glucose monitoring", APPLIED SPECTROSCOPY, vol. 60, no. 4, 2 April 2006 (2006-04-02), pages 441-449, XP55272652, US ISSN: 0003-7028, DOI: 10.1366/000370206776593780 * section "Methodology for creating a calibration model" and figure 2 * * section "Characteristics of the calibration model" * ----- -/-- | 1-7 | TECHNICAL FIELDS SEARCHED (IPC) G06F A61B G01N G06K |

The present search report has been drawn up for all claims

| Place of search | Date of completion of the search | Examiner |
|---|---|---|
| Berlin | 13 May 2016 | Domingo Vecchioni, M |

EPO FORM 1503 03.82 (P04C01)

Europäisches
Patentamt

European
Patent Office

Office européen
des brevets

## EUROPEAN SEARCH REPORT

Application Number

EP 15 18 8542

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GOLIC M ET AL: "Short-wavelength near-infrared spectra of sucrose, glucose, and fructose with respect to sugar concentration and temperature", APPLIED SPECTROSCOPY, vol. 57, no. 2, February 2003 (2003-02), pages 139-145, XP55272663, US ISSN: 0003-7028, DOI: 10.1366/000370203321535033 * section "Introduction", paragraphs 1-3 * ----- | 1-7 | |
| A | EP 0 869 348 A2 (MATSUSHITA ELECTRIC WORKS LTD [JP]) 7 October 1998 (1998-10-07) * abstract * ----- | 1-7 | |

TECHNICAL FIELDS
SEARCHED        (IPC)

The present search report has been drawn up for all claims

| Place of search | Date of completion of the search | Examiner |
|---|---|---|
| Berlin | 13 May 2016 | Domingo Vecchioni, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

                                     

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 15 18 8542

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2016

| Patent document cited in search report | | Publication date | Patent family member(s) | | Publication date |
|---|---|---|---|---|---|
| EP 2784484 | A1 | 01-10-2014 | EP | 2784484 A1 | 01-10-2014 |
| | | | JP | 2014190795 A | 06-10-2014 |
| | | | US | 2014297197 A1 | 02-10-2014 |
| EP 0869348 | A2 | 07-10-1998 | CN | 1201905 A | 16-12-1998 |
| | | | DE | 69830629 D1 | 28-07-2005 |
| | | | DE | 69830629 T2 | 11-05-2006 |
| | | | EP | 0869348 A2 | 07-10-1998 |
| | | | JP | 4936203 B2 | 23-05-2012 |
| | | | JP | 2010066280 A | 25-03-2010 |
| | | | US | 5957841 A | 28-09-1999 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010066280 A

- JP 2001099710 A

**Non-patent literature cited in the description**

- **ZENG-PING CHEN ; JULIAN MORRIS ; ELAINE MARTIN.** *Extracting Chemical Information from Spectral Data with Multiplicative Light Scattering Effects by Optical Path-Length Estimation and Correction,* 2006
- **AAPO HYVARINEN ; JUHA KARHUMEN ; ERKKI OJA.** Independent Component Analysis. John Wiley & Sons, Inc, 2001

- Independent Component Analysis. department of Tokyo Denkki University, February 2005
- Independent Component Analysis. publishing department of Tokyo Denkki University, February 2005
- **MINAMI MIHOKO ; SHINTO EGUCHI.** *Robust Blind Source Separation by β-Divergence,* 2002
- Independent component analysis for noisy data?. *MEG data analysis,* 2000